# EUROPEAN PATENT SPECIFICATION

(11) **EP 3 532 036 B1**
(45) Date of publication and mention of the grant of the patent: **29.12.2021**
(21) Application number: 17811376.7
(22) Date of filing: 24.10.2017
(51) Int. Cl.: A61K 9/19, A61K 9/51, A61K 35/12, A61K 35/28

(54) **PROCESS FOR ISOLATING AND LYOPHILIZING EXTRACELLULAR VESICLES**
VERFAHREN ZUR ISOLIERUNG UND LYOPHILISIERUNG VON EXTRAZELLULÄREN VESIKELN
PROCÉDÉ D'ISOLEMENT ET DE LYOPHILISATION DE VÉSICULES EXTRACELLULAIRES

(30) Priority: 28.10.2016 IT 201600109148
(43) Date of publication of application: 04.09.2019
(73) Proprietor: Pharmaexceed S.r.l., 27100 Pavia (IT)
(72) Inventor: PERTEGHELLA, Sara, I-27010 Cura Carpignano (PV) (IT); BARI, Elia, I-22010 San Nazzaro Val Cavargna (CO) (IT); CHLAPANIDAS, Theodora, 27058 Voghera (PV) (IT); SORLINI, Marzio, I-22070 Bregnano (CO) (IT); DE GIROLAMO, Laura, I-27100 Pavia (IT); PERUCCA ORFEI, Carlotta, I-27100 Pavia (IT); VIGANÒ, Marco, I-27100 Pavia (IT); TORRE, Maria Luisa, I-27100 Pavia (IT)
(74) Representative: Cernuzzi, Daniele
(86) International application number: PCT/IB2017/056591
(87) International publication number: WO 2018/078524

(56) References cited:
- EP-A1- 2 687 219
- WO-A1-2016/090183
- WO-A1-2018/070939
- CN-A- 104 488 850
- MASSIMO FAUSTINI ET AL: "Nonexpanded Mesenchymal Stem Cells for Regenerative Medicine: Yield in Stromal Vascular Fraction from Adipose Tissues", TISSUE ENGINEERING PART C: METHODS, vol. 16, no. 6, 1 December 2010 (2010-12-01), pages 1515-1521, XP055071049, ISSN: 1937-3384, DOI: 10.1089/ten.tec.2010.0214 cited in the application
- Richard J Lobb ET AL: "Optimized exosome isolation protocol for cell culture supernatant and human plasma", Journal of extracellular vesicles, 1 January 2015 (2015-01-01), pages 27031-11, XP055279331, Sweden DOI: 10.3402/jev.v4.27031 Retrieved from the Internet: URL:http://www.ncbi.nlm.nih.gov/pmc/articl es/PMC4507751/pdf/JEV-4-27031.pdf [retrieved on 2016-06-09]
- DINH HA ET AL: "Exosomes as therapeutic drug carriers and delivery vehicles across biological membranes: current perspectives and future challenges", PROTECTION BY THE GROSS SAPONINS OF TRIBULUS TERRESTRIS AGAINST CEREBRAL ISCHEMIC INJURY IN RATS INVOLVES THE NF-[KAPPA]B PATHWAY, vol. 6, no. 4, 1 July 2016 (2016-07-01), pages 287-296, XP055303644, ISSN: 2211-3835, DOI: 10.1016/j.apsb.2016.02.001
- ALI H. ALHASAN ET AL: "Exosome Encased Spherical Nucleic Acid Gold Nanoparticle Conjugates as Potent MicroRNA Regulation Agents", SMALL, vol. 10, no. 1, 17 September 2013 (2013-09-17), pages 186-192, XP055268134, DE ISSN: 1613-6810, DOI: 10.1002/smll.201302143
- FUHRMANN GREGOR ET AL: "Stability of extracellular vesicles during lyophilization - implications for their pharmaceutical use", JOURNAL OF EXTRACELLULAR VESICLES, , vol. 5, no. 1 23 February 2016 (2016-02-23), page 14, XP009514159, ISSN: 2001-3078, DOI: 10.3402/JEV.V5.30924 Retrieved from the Internet: URL:1077952576 [retrieved on 2020-11-03]
- BARI ET AL: "Adipose Mesenchymal Extracellular Vesicles as Alpha-1-Antitrypsin Physiological Delivery Systems for Lung Regeneration", CELLS, vol. 8, no. 9, 23 August 2019 (2019-08-23) , page 965, XP055786126, DOI: 10.3390/cells8090965
- BARI ELIA ET AL: "Pilot Production of Mesenchymal Stem/Stromal Freeze-Dried Secretome for Cell-Free Regenerative Nanomedicine: A Validated GMP-Compliant Process", CELLS, vol. 7, no. 11, 30 October 2018 (2018-10-30), page 190, XP055786111, DOI: 10.3390/cells7110190

## Description

### FIELD OF THE INVENTION

The present invention relates to a process for isolating and lyophilizing extracellular vesicles present in biological fluids. In particular, the invention relates to a combined process of dialysis or ultrafiltration and lyophilization of biological fluids comprising microvesicles and/or exosomes which allows obtaining a powder product containing microvesicles and/or exosomes. Moreover, according to a preferred aspect, the invention relates to a process for obtaining a powder product of microvesicles and/or exosomes in turn containing nanoparticles loaded with one or more biologically active substances.

### PRIOR ART

Cells communicate and exchange information in two fundamental manners: through direct contact between cells and through the secretion of biologically active substances (paracrine mechanisms); the product of cellular secretion as a whole can be referred to as secretome. Secretome consists of soluble factors (such as cytokines) and insoluble structures such as exosomes and microvesicles.

Exosomes and microvesicles are nanometric particles with an average diameter generally between 30 and 1000 nm, delimited by a generally two-layered lipid membrane.

Microvesicles and exosomes contain biomolecules, including proteins, messenger RNA and micro RNA and, due to their composition, they have been proposed as innovative therapy as an alternative to the source cells as they have many advantages, including a minor propensity to stimulate the immune system, decreasing the risk of tumorigenesis, the ability to cross the blood-brain barrier, a minor tendency to vascular obstruction.

Secretome has been proposed in regenerative medicine therapies for the treatment of various diseases such as arthrosis, cardiovascular diseases, neurological diseases, lung disease, diabetes and many other (Lener T., et al., "Applying extracellular vesicles based therapeutics in clinical trials - an ISEV position paper", J Extracell Vesicles., Vol. 4, 2015, 30087). In studies reported in the literature, the success rate and the duration of the effect were significantly inconsistent, mainly due to the sometimes insufficient amount of biologically active substances (proteins, glycoproteins, nucleic acids) that reach unchanged the site of action.

Although to date there is still no specific legislation, secretome, inclusive of microvesicles and exosomes, can be considered as a biotechnological drug and preparations must be established for clinical use which meet the Good Manufacturing Practice (GMP) in order to ensure safety and therapeutic efficacy, by controlling the quality of the product (such as, but not limited to, quantitative/qualitative uniformity) and of the production process (such as, but not limited to, robustness and reproducibility).

Various methods have been described for the isolation of microvesicles and/or exosomes, but all on a laboratory scale only and to date, none has been implemented on an industrial/semi-industrial scale following the GMP.

The method most commonly used is ultracentrifugation, in which bodily fluids are subjected to centrifugation at high speed. However, the yield of microvesicles and/or exosomes as well as the quality of the final product may be influenced by the properties of the instrument (e.g., type of rotor, rotor sedimentation angle and/or centrifugal force radius) and by the operator. Moreover, ultracentrifugation may cause the aggregation or rupture of the microvesicles and/or exosomes due to high centrifugal forces, thus losing the integrity of the vesicular structures.

New isolation techniques have been investigated for the large-scale manufacturing of microvesicles and/or exosomes, such as molecular exclusion chromatography and tangential flow filtration. Recently, some researchers have shown that the combination of ultrafiltration and molecular exclusion chromatography allows a significantly higher yield of microvesicles and/or exosomes to be obtained compared to ultracentrifugation, without changing the biophysical properties and the protein content of the source material (Nordin JZ, et al., "Ultrafiltration with size-exclusion liquid chromatography for high yield isolation of extracellular vesicles preserving intact biophysical and functional properties", Nanomedicine, Vol. 11, Issue 4, May 2015, 879-883).

Isolation techniques of microvesicles and/or exosomes were described in recently published patents and patent applications, such as US9347087, US8901284, US9005888, WO2016033695, WO2016022654, WO2015131153, and WO2012087241.

In particular, WO2016090183 discloses processes for producing stable exosome formulations in lyophilized form, for example by ultrafiltration and diafiltration.

CN104488850 discloses a method for preparing an exosome freeze-dried powder of human amniotic mesenchymal stem cells: the method comprises the following steps: mixing trehalose with exosomes of the human amniotic mesenchymal stem cells as a cryoprotectant; filtering and removing bacteria by virtue of a filter membrane; and firstly carrying out ultralow temperature pre-freezing, and then carrying out low-temperature freezing at a certain vacuum degree.

M. Faustini et al, "Non-expanded mesenchymal stem cells for regenerative medicine: yield in stromal vascular fraction from adipose tissues", Tissue Engineering, 2010, Vol. 16, Issue 6, 1515-21, discuss the optimization of the adipose SVF-based therapy, and the effect of the collection site, surgical procedure, and tissue processing techniques on SVF yield. The paper also discloses methods for recovering adipose tissue samples, which are then digested in different condition with collagenase.

Another problem known in the art consists in the preservation of microvesicles and/or exosomes. To date, the only method of preservation of microvesicles and/or exosomes is cryopreservation at a temperature equal to or lower than -20°C, in absence of cryo-protectors (Zhou H, et al., "Collection, storage, preservation, and normalization of human urinary exosomes for biomarker discovery", Kidney Int., Vol. 69, Issue 8, 2006, 1471-1476). The lack of cryo-preservatives is a considerable limitation since the water crystals formed during the freezing process easily break the biological membranes that make up the microvesicles and exosomes. In addition, the marked osmotic stress the secretome is subjected to during freezing and thawing can irreversibly damage the membranes, the cargo contained therein, as well as membrane proteins, adhesion and homing molecules and receptors (Ohno S, et al., "Focus on extracellular vesicles: development of extracellular vesicle-based therapeutic systems", Int. J. Mol. Sci., Vol. 17, 2016, 172).

Therefore, despite the progress made, there is still a need to develop a process of isolation and preservation of microvesicles and/or exosomes that is feasible and economically viable in GMP, easily scalable, reproducible and, above all, a pharmaceutical preparation stable over time.

### SUMMARY OF THE INVENTION

The Applicant has found a process of isolation and preservation of microvesicles and/or exosomes that overcomes the drawbacks of the methods described in the literature.

In particular, the Applicant has optimized a combined process of dialysis or ultrafiltration and lyophilization applicable to biological fluids comprising microvesicles and/or exosomes (like the supernatant of cultured cells or bodily fluids) which allows a solid material in the form of lyophilized powder to be obtained.

The lyophilized powder obtained by the process of the invention can be easily made in suitable premises for the pharmaceutical production of sterile products or can be packaged and made sterile (for example, but not limited to, using radiation), obtaining a finished solid pharmaceutical product (ready to use or intended for extemporaneous preparation) or a semi-finished product for the preparation of solid, liquid or semisolid pharmaceutical forms (solutions, suspensions, emulsions, creams, ointments, pastes or gels, granules, capsules, tablets) for the administration of microvesicles and/or exosomes.

The lyophilized powder obtained by the process of the invention can be easily characterized by following procedures certifiable in GMP, such as (I) titration in proteins and phospholipids, (II) size distribution of microvesicles or exosomes, (III) in vitro cytotoxicity, (IV) in vitro biological activity (immunomodulation and anti-inflammatory activity).

The Applicant noted that by carrying out the dialysis or ultrafiltration in the presence of particular buffer solutions, a greater concentration of microvesicles and/or exosomes could be obtained while preserving the integrity thereof.

The Applicant also noted that by carrying out the lyophilization in the presence of a suitable cryo-protective agent it was possible to obtain a solid and stable product with a high protein content, while devoid of cytotoxicity and effective in reducing inflammation in a cellular model in vitro, by stimulating articular chondrocytes.

The Applicant finally noted that the addition of nanoparticles loaded with a biologically active substance during the cell culture step allowed cells loaded with such nanoparticles to be initially obtained, and thus microvesicles and/or exosomes in turn loaded with such nanoparticles.

Therefore, a first aspect of the present invention relates to a production process of microvesicles and/or exosomes as defined by claim 1, characterized in that it comprises the following steps:
(i) collecting a biological fluid comprising microvesicles and/or exosomes,
(ii) dialyzing or ultrafiltering said biological fluid using a membrane having a threshold value (cut-off) equal to or smaller than 5000 Dalton and a dialysis or ultrafiltration fluid,
(iii) adding a cryo-protector to the dialyzed or ultrafiltered solution obtained from step (ii), and
(iv) lyophilizing the solution resulting from step (iii) obtaining a lyophilized powder comprising microvesicles and/or exosomes in an amount equal to or greater than 20% w/w with respect to the total weight of said lyophilized powder; and at least one cryo-protector in an amount equal to or smaller than 80% w/w.

Preferably, the biological fluid is the supernatant of cultured cells, advantageously stem cells or a bodily fluid, such as, but not limited to, plasma, serum or amniotic fluid.

According to a preferred aspect, the supernatant is collected from cultured cells in a culture medium for at least 4 hours.

According to a further preferred aspect, the cells are cultured in a culture medium containing nanoparticles loaded with a biologically active substance.

In a second aspect, the present invention relates to a product in the form of lyophilized powder obtained by the above process, as defined by claim 13.

According to a preferred aspect, the microvesicles and/or exosomes comprise nanoparticles loaded with a biologically active substance.

### BRIEF DESCRIPTION OF THE FIGURES

Figure 1 shows the graph of the size distribution of the microvesicles and/or exosomes described in example 4.
Figure 2 shows a SEM photograph (12,000x) of the microvesicles and/or exosomes described in example 4.
Figures 3a and 3b show two TEM photographs of the microvesicles and/or exosomes described in example 4.
Figure 4 shows the results of the immunomodulation assay described in example 5.
Figure 5 shows the results of the uptake assay of nanoparticles described in example 6.
Figure 6 shows the average scores of acute inflammation and respective standard deviation at 3, 7 and 21 days shown in table 8 of example 9.
Figure 7 shows the average scores of chronic inflammation and respective standard deviation at 3, 7 and 21 days shown in table 8 of example 9.
Figure 8 shows the average scores of the deposition of granulation tissue and relative standard deviation at 3, 7 and 21 days shown in table 8 of example 9.

### DETAILED DESCRIPTION OF THE INVENTION

The term "microvesicles and/or exosomes" means particles surrounded by a single- or two-layered lipid membrane having a size of between 20 nm and 1,000 nm secreted by cells and present in a biological fluid.

The term "biological fluid" means a supernatant of cultured cells or a bodily fluid.

The term "minimal culture medium" means a culture medium comprising the minimum nutrients possible where such nutrients, generally limited to a carbon energy source (e.g. sugars) and some salts, are defined in both qualitative and quantitative terms, and lacking serum.

The term "disorder" refers to any alteration of the normal function of an organ, apparatus or system of a living animal or human being caused by external pathogenic and/or traumatic agents, by internal agents and/or by degenerative phenomena caused by ageing.

The microvesicles and/or exosomes may be present in different bodily fluids (such as, but not limited to, plasma, saliva, urine, serum, synovial fluid, follicular fluid, amniotic fluid, cerebrospinal fluid) or may be present in the culture supernatant of different cell lines (such as, but not limited to, adipocytes, astrocytes, beta cells, lymphocytes, endothelial cells, cancer cells, cardiomyocytes, dendritic cells, stem cells, epithelial cells, erythrocytes, liver cells, keratinocytes, macrophages, monocytes, oligodendrocytes, platelets, lymphocytes T).

The biological fluid preferably used in the process of the present invention is represented by the supernatant of cultured cells, particularly of stem cells. The cells useful in the present invention are preferably mesenchymal stem cells.

Mesenchymal stem cells (MSC) originate from mesoderm, the germ layer of blastocyst between ectoderm and endoderm. MSC are self-renewable multipotent stem cells able to differentiate into different cell lines, among which are osteoblasts, chondrocytes, myocytes and adipocytes, originating various cellular tissues such as bones, cartilage, tendons, muscle and adipose tissue. MSC are spread throughout the body in mammals and are responsible for tissue regeneration. The isolation of MSC has been performed from multiple tissue districts, including bone marrow, umbilical cord and recently and to an increasing extent, from the adipose tissue, which contains a large amount of MSC.

Besides their capacity for differentiation, MSC have been shown to modulate the immune response and inflammation and promote tissue healing and protective mechanisms, making them usable in the treatment of various diseases (Caplan AI, "What's in a name?" Tissue Engineering Part A. August 2010, Vol. 16, No. 8: 2415-2417). MCS-based drugs subjected to important manipulation are referred to as advanced therapy medicinal products.

MSC can be isolated from the bone marrow stromal component (where they represent about 0.01% of all nucleated cells), from umbilical cord blood, the from the placenta, from peripheral blood and the adipose tissue (fat tissue).

Preferably, MSC are isolated from adipose tissue. The adipose tissue contains a stromal vascular fraction from which mesenchymal stem cells derived from adipose tissue can be isolated (AD-MSC). AD-MCS have phenotype and gene expression profile similar to that of bone marrow stem cells. AD-MSC have the capacity for self-renewal and growth in the long term and are able to differentiate into different cell types such as adipocytes, osteoblasts and chondrocytes.

AD-MSC are isolated from the adipose tissue by washing with buffer solution and enzymatic digestion.

Washing and/or digestion may be carried out using any buffer solution known in the art such as, for example, phosphate-buffered saline (PBS), borate buffered saline (BBS) and TRIS buffered saline (TBS-TRIS).

Digestion may be carried out by any enzyme known in the art able to degrade and progressively separate cells from the extracellular matrix by breaking the peptide bonds present in collagen. Typically, collagenases are used for this operation such as AFA collagenase, type I collagenase, type II collagenase, type III collagenase, type IV collagenase and CLSPA collagenase.

Advantageously, digestion is carried out in the presence of antibiotics such as penicillin, streptomycin and amphotericin, in a concentration of less than 5% w/v, preferably less than 3% w/v.

The stromal vascular fraction, containing MSC, can be separated from the digestion medium by filtration and/or centrifugation, and then placed in culture medium, preferably in single-layer conditions, up to achieving sub-confluence.

Cell culture is performed in minimal culture medium adhesion plates such as DMEM (Dulbecco's modified Eagle's Medium), IMDM (Iscove's Modified Dulbecco's Media), Ham's F10, Ham's F12, MEM (Minimal Essential Medium), G-MEM (Glasgow Minimal Essential Medium), BME (Basal Medium Eagle), or mixed media such as DMEM/Ham's F12 (1:1 ratio), Advanced DMEM/Ham's F12 and IMDM/MEM.

Preferably, the minimal culture medium is supplemented with serum of animal origin such as fetal bovine serum (FBS), fetal calf serum (FCS), or commercially available artificial serum substitutes, such as Knockout^{™} SR serum (ThermoFisher Scientific, Waltham, MA, USA), X-Vivo^{™}10 and X-Vivo^{™}20 (Lonza Walkersville, Inc, Walkersville, MD, USA), Lipumin^{™} 10× and SerEx 10x (PAA Laboratories GmbH, Pasching, Austria), SR3 (Sigma, St Louis, MO, USA), serum substitute supplement (SSS) (Irvine Scientific, Santa Ana, CA, USA) and Plasmanate (Bayer Healthcare, West Haven, CT, USA) or commercially available platelet lysate (Lyset^{™}, Carlo Erba Reagents, Italy).

MSC are then treated with a cell detachment solution, typically trypsin-EDTA , and plated again at 37°C and in a 5% carbon dioxide atmosphere in single-layer conditions. The process (cellular expansion) is repeated several times, preferably from 1 to 12 times, more preferably from 1 to 3 times.

Adhering MSC are then cultured in a minimal culture medium for at least 4 hours, preferably at least 12 hours, and preferably at least 18 hours. According to a more preferred aspect, the culture takes place for less than 96 hours, preferably less than 48 hours. Advantageously, the culture takes place for about 24 hours.

The Applicant noted that, under these conditions, MSC produce a considerable and reproducible amount of microvesicles and/or exosomes that are released into the culture medium.

The Applicant further noted that the addition of nanoparticles loaded with a biologically active substance during the MSC culture step allowed cells loaded with such nanoparticles to be initially obtained, and then, after culture in minimal medium, microvesicles and/or exosomes in turn loaded with such nanoparticles.

The culture supernatant, containing microvesicles and/or exosomes, is collected and the MSC are again treated with a cell detachment solution, typically trypsin-EDTA.

In the process of the present invention, the biological fluid (supernatant or bodily fluid) is subjected to a dialysis or ultrafiltration process using a membrane having a threshold value (cut-off) equal to or smaller than 5000 Dalton.

Dialysis membranes are commercially available from several manufacturers, such as the membranes Spectra/Por^{®} (Spectrum Laboratories, Inc, Rancho Dominguez, CA USA), Visking (Medicell Membranes LTD, London, UK), SnakeSkin^{®} (TermoFisher Scientific, Rockford, IL, USA).

Ultrafiltration equipment is commercially available from the same manufacturer, such as for example the TFF (Tangential Flow Filtration) ultrafiltration system from Spectrum Laboratories or the ultrafiltration units for centrifugation Amicon^{™} (Merk Millipore).

The biological fluid (supernatant or bodily fluid) is enclosed within a container (dialysis bag or tube) made with the membrane having pores of size equal to or smaller than 5000 Dalton, and the container is placed in a suitable ultrafiltration or dialysis fluid.

The ultrafiltration or dialysis fluid may be water or buffer solution with a concentration of salts lower than the concentration of salts of the biological fluid, in other words with an osmotic pressure lower than the osmotic pressure of the biological fluid.

Preferably, the buffer solution is selected from the group comprising inorganic salt solutions, such as sodium phosphate, sodium chloride, sodium acetate, sodium citrate, or organic salt solutions, such as tromethamine hydrochloride (HCI-TRIS), MOPS, MES, HEPES and TES. Buffer mixtures such as phosphate buffered saline (PBS) comprising sodium and potassium chloride, disodium phosphate and monopotassium phosphate may advantageously be used.

Examples of buffer solutions useful in the process of the present invention is a solution of Na₂HPO₄ * 7H₂0 (10mM), a solution of tromethamine hydrochloride (TRIZMA 1.28M), a solution of NaCl (0.54M) and PBS lacking Ca²⁺ and Mg²⁺ ions.

Advantageously, the dialysis fluid comprises polyoxyalkylene having an average molecular weight equal to or greater than 100 g/mol.

Preferably, the polyoxyalkylene is a polyethylene glycol (PEG) having an average molecular weight equal to or greater than 100 g/mol, preferably equal to or greater than 300 g/mol, more preferably equal to or greater than 500 g/mol.

Preferably, the polyoxyalkylene useful in the present invention is a polyethylene glycol (PEG) having an average molecular weight equal to or smaller than 4000 g/mol, preferably equal to or smaller than 3000 g/mol, more preferably equal to or smaller than 2500 g/mol.

Advantageously, the polyoxyalkylene useful in the present invention is a polyethylene glycol (PEG) with an average molecular weight between 1000 and 2000 g/mol.

The solution obtained by the ultrafiltration or dialysis process comprises an increased concentration of microvesicles and/or exosomes and a low salt and/or impurity content. The dialyzed or ultrafiltered solution is then subjected to a lyophilization process by means of conventional lyophilization techniques to provide a powder material which is adapted for prolonged storage at room temperature or at lower temperatures.

According to the process of the present invention, the dialyzed solution or ultrafiltered solution is supplemented with a cryo-protector.

The cryo-protector is typically represented by a sugar, amino acids, polyols or mixtures thereof. Examples of sugars useful as cryo-protectors in the process of the present invention are: mannitol, lactose, sucrose, trehalose, sorbitol, glucose, maltose, fructose, and raffinose. Examples of amino acids useful as cryo-protectors in the process of the present invention are: arginine, alanine, glycine and histidine. Examples of polyols useful as cryo-protectors in the process of the present invention are: ethylene glycol, 1,3-propanediol, glycerol and cyclo-dextrins. Preferably, mannitol, glycine and mixtures thereof are used as cryo-protectors in the present invention. The addition of cryo-protector can be conducted by dissolving the cryo-protector directly into the dialyzed or ultrafiltered supernatant or by adding an aqueous solution of cryo-protector previously prepared.

Advantageously, the cryo-protector or the mixture of cryo-protectors are added to the dialyzed or ultrafiltered solution in amounts of between 0.25 and 25% w/v, preferably between 0.5 and 10% w/v, more preferably between 0.5 and 5% w/v.

The freezing of the dialyzed or ultrafiltered solution takes place at temperatures below -50°C, preferably below -70°C, with the aid of dry ice or liquid nitrogen. The dialyzed or ultrafiltered and frozen solution is transferred into a lyophilizer, where vacuum is applied to lower the atmospheric pressure and allow sublimation of the ice (i.e., the transition of water from the solid phase to the vapor phase without passing through the liquid phase).

Lyophilizing equipment (lyophilizers) is widely available on the market. A lyophilizer essentially consists of a drying chamber, a condenser to collect the sublimated vapor from the product, a cooling system for the drying chamber and the condenser, and a vacuum pump to reduce the pressure in the drying chamber and the condenser.

Lyophilization is advantageous as it provides a product in powder form, stable and ready for packaging or already packaged, containing microvesicles and/or exosomes devoid of residual cells or microbes.

The process of the present invention allows obtaining a product in the form of lyophilized powder comprising microvesicles and/or exosomes having a diameter between 20 and 5000 nm and an average diameter between 50 and 200 nm.

The content of microvesicles and/or exosomes in the lyophilized powder of the present invention consists of at least 20% w/w with respect to the total weight of the lyophilized powder, preferably at least 30% w/w with respect to the total weight of the lyophilized powder, and more preferably at least 40% w/w with respect to the total weight of the lyophilized powder. The remaining content equal to or smaller than 80% w/w, preferably equal to or smaller than 70% w/w, more preferably equal to or smaller than 60% w/w, is represented by the cryo-protector or mixture of cryo-protectors.

Preferably, the lyophilized powder of the present invention contains microvesicles and/or exosomes in an amount of between 20% and 60% w/w, more preferably between 25% and 55% w/w, and cryo-protector or a mixture of cryo-protectors in an amount of between 80% and 40% w/w, more preferably between 75% and 45% w/w.

According to a preferred aspect of the invention, the microvesicles and/or exosomes comprise nanoparticles loaded with a biologically active substance.

Like the MSC, the product obtained according to the present invention may be useful in regenerative medicine therapies, for the treatment of various diseases such as (but not limited to) arthrosis, cardiovascular diseases, such as heart failure, respiratory diseases, neurological diseases, such as Parkinson's or Alzheimer's disease, pain, such as chronic and/or neuropathic pain, bone marrow failure, diabetes and cancer, in aesthetic medicine treatments of soft tissues and skin anti-aging cosmetics.

The product obtained according to the present invention has been shown to be particularly useful in the treatment of acute and chronic wounds and injuries in an animal model, as described in example 9.

At the same time, the introduction of nanoparticles loaded with biologically active substances allows directing by means of microvesicles and/or exosomes directly towards the organ and/or apparatus concerned by the effect of the biologically active substance.

Organs and/or apparatuses mainly concerned comprise, for example (but not limited to), those of the cardiovascular system, respiratory system, gastrointestinal system, integumentary system, muscular system, skeletal system and central and peripheral nervous system.

The choice of the biologically active substance is not particularly limited. Any substance capable of being loaded into nanoparticles and effective in a disorder of one of the systems mentioned above may be comprised in the microvesicles and/or exosomes obtained by the process of the present invention.

By way of example, there may be mentioned cardiovascular drugs (such as antihypertensives, antiarrhythmic drugs, diuretics), drugs of the central and peripheral nervous system (such as anesthetics, hypnotics, sedatives, anxiolytics, anti-Parkinson's drugs), painkilling drugs (such as anti-inflammatory and analgesic drugs), antimicrobial drugs (such as sulfamidic drugs, antibiotics, antivirals and antifungal drugs), chemotherapy drugs, anti-tumor drugs, anti-tumor immunotherapy drugs, vaccines (such as tumor vaccines or influenza vaccines), hormones, peptide and protein drugs, vitamins, plant extracts and phytocomplexes.

The product comprising microvesicles and/or exosomes obtained by the process according to the present invention may be administered to a living animal or human being by any suitable administration route, such as parenteral, inhalation, topical, ophthalmic, auricular, and transmucosal (such as, but not only, rectal, vaginal and buccal).

In the case of parenteral administration, the product is reconstituted by the addition to a suitable injectable saline and administered parenterally, for example subcutaneously or venous, through injection, infusion, catheter or intrathecal infuser. In the case of administration by the respiratory route, the product can be formulated in the form of powder for inhalation or solubilized for aerosols. In the case of administration by ophthalmic, auricular and transmucosal route, the product can be formulated in the form of a solution, ointment, gel or cream. In the case of topical administration also for the treatment of wounds and skin ulcers, the product can be formulated in the form of a membrane for advanced dressings, for example made from sodium alginate, calcium alginate, cellulose derivatives (such as carboxymethylcellulose), esters of hyaluronic acid, silk proteins (such as fibroin and sericin), non-woven fabric, and so on.

In the case of cosmetic use, the product can be formulated in the form of ointment, cream, cream-gel, gel, lotion, paste, or solution for topical or ophthalmic application or for irrigation.

The product comprising microvesicles and/or exosomes obtained by the process according to the present invention can be easily packaged and made sterile (such as, but not only, by irradiation as described in Sakar F. et al., "Nano drug delivery systems and gamma radiation sterilization", Pharmaceutical Development and Technology), obtaining a solid pharmaceutical product for the administration of microvesicles and/or exosomes.

The present invention will be further understood with reference to the following non-limiting examples.

### Example 1

Isolation of mesenchymal stem cells (MSC) from human adipose tissue

The adipose tissues were obtained from informed donors. The samples were repeatedly washed with phosphate buffered saline (PBS) free of calcium ions (Ca²⁺) and magnesium (Mg²⁺) (Euroclone, Milan, Italy) and finely minced with surgical scissors. Tissue digestion was performed in a bath set at 37°C provided with stirrer using the type II collagenase enzyme (Sigma-Aldrich, Milan, Italy) dissolved in PBS containing calcium ions (Ca²⁺) and magnesium (Mg²⁺) (Euroclone, Milan, Italy), supplemented with 1% penicillin/streptomycin (Euroclone, Milan, Italy) and 1% amphotericin B (Euroclone).

After one hour, to the cell suspension were added the DMEM medium (Dulbecco's modified Eagle's Medium) and Ham's F12 medium in a 1:1 ratio (Euroclone, Milan, Italy) supplemented with 10% fetal bovine serum (FBS, Gibco, Thermo Fisher Scientific, Milan, Italy).

The digested tissue was filtered through a 70µm cellular sieve (Greiner Bio-One, Milan, Italy) and centrifuged at 600 g for 5 minutes (M. Faustini et al, "Non-expanded mesenchymal stem cells for regenerative medicine: yield in stromal vascular fraction from adipose tissues", Tissue Engineering, 2010, Vol. 16, Issue 6, 1515-21). The stromal vascular fraction thus recovered was cultured in monolayer conditions (100,000 cells/cm²) in DMEM/F12 medium in 1:1 ratio supplemented with 10% fetal bovine serum, 1% penicillin/streptomycin and 1% amphotericin B.

Upon reaching the sub-confluence, the MSC were removed by treatment with 0.05% trypsin-EDTA (Euroclone, Milan, Italy) and seeded back into the flask in monolayer conditions (10,000 cells/cm²) at 37°C in a 5% CO₂ atmosphere.

### Isolation and lyophilization of microvesicles and/or exosomes

When the MSC reached sub-confluence, they were transferred and cultured in DMEM/F12 minimal medium for 4 or 24 hours. Then, the supernatant was collected and the MSC were removed by treatment with 0.05% trypsin-EDTA (Euroclone, Milan, Italy) to assess the number of cells and their viability. The supernatant was dialyzed for three days using dialysis membranes having a cut-off of 3500 Dalton (Spectra/Por, Spectrum Laboratories, Milan, Italy) in the presence of different types of buffer solutions (ultrapure distilled water, 10mM Na₂HPO₄ * 7H₂0, 1.28M TRIZMA, 0.54M NaCl and PBS devoid of Ca²⁺ and Mg²⁺ ions) as shown in the following tables of examples 2 and 3.

To concentrate the product, the dialysis process was conducted in the presence of a buffer solution containing 5% w/v polyethylene glycol (PEG 1500, Merck Millipore, Milan, Italy).

The dialyzed product was supplemented with a cryo-protector agent (glycine, mannitol) alone or in combination, in the amounts shown in the following tables of examples 2 and 3, frozen at -20°C, and then subjected to the lyophilization process with vacuum below 1.33 mbar.

### Example 2

The samples obtained according to the procedure of example 1 were subjected to the BCA assay with the Protein Assay Kit from Thermo Scientific (Milan, Italy) for the quantification of the proteins contained in the dialyzed and lyophilized product, and the MTT assay to assess the cytotoxicity on human fibroblasts.

### BCA assay

The total protein content of the dialyzed and lyophilized samples of microvesicles and/or exosomes obtained with the procedure of example 1 using the buffer and the cryo-protector of the following table 1 was determined using the BCA-Protein Assay kit (Thermo Scientific, Milan, Italy), according to the manufacturer's specifications.

The absorbance-concentration calibration curve was generated using standard bovine serum albumin (BSA, Sigma-Aldrich). The working reagent solution was added to each sample or standard (1:1 ratio), and the sets were then incubated at 37°C for 2 hours before measuring the absorbance at 562 nm with a microplate reader (Synergy HT, BioTek, United Kingdom). The protein concentration was extrapolated from a concentration graph against absorbance obtained from standard protein solutions, using a third degree polynomial equation, with r² 0.99 for each assay.

The results are summarized in the following table 1.

**TABLE 1**

| **Sample** | **Incubation in minimal medium (hours)** | **Dialysis buffer** | **Glycine (% w/v)** | **Mannitol (% w/v)** | **Protein (µg/ml)** | **St. D.** |
|---|---|---|---|---|---|---|
| 1A | 4 | Na₂HPO₄ | 2 | 0 | 68.5 | 39.58 |
| 1B | 24 | Na₂HPO₄ | 2 | 0 | 14.9 | 0.86 |
| 1C | 24 | NaCl | 0.2 | 0 | 66.6 | 3.15 |
| 1D | 24 | NaCl | 0 | 3 | 34.4 | 2.34 |
| 1E | 24 | NaCl | 0.75 | 3 | 25.6 | 3.12 |
| 1F | 24 | TRIZMA | 0.75 | 3 | 6.6 | 0.56 |
| 1G | 24 | PBS | 0.75 | 3 | 24.5 | 6.68 |
| 1H | 24 | H₂O | 0.75 | 3 | 42.3 | 5.98 |
| 1I | 24 | Na₂HPO₄ | 0.75 | 3 | 17.1 | 0.35 |

The results in table 1 showed that the culturing time of the MSC in the minimal medium influenced, with the same buffer and cryo-protective agent, the amount of resulting proteins, although the data obtained at 4 hours showed values with a high dispersion, symptom of high variability between the samples analyzed.

With the same type and amount of cryo-protective agent, the amount of resulting proteins is influenced by the type of buffer used, with higher values for the NaCl and PBS buffer and lower values for the Na₂HPO₄ and TRIZMA buffer. The use of water surprisingly showed the best value.

With the same buffer, using NaCl, mannitol alone gave better results than the glycine-mannitol combination.

### MTT assay

Human fibroblasts were seeded (10,000 cells/cm²) in a 96-well plate (Greiner Bio-One, Milan, Italy) and cultured for 24 hours at 37°C under 5% CO₂ atmosphere, in DMEM HG (high glucose) medium supplemented with 10% fetal bovine serum, 1% penicillin/streptomycin and 1% amphotericin B.

The dialyzed and lyophilized samples of microvesicles and/or exosomes obtained with the procedure of example 1 using the buffer and the cryo-protector of the following table 2 were incubated with the cells at three concentrations (2.5, 1.25 and 0.6 mg/well) for 24 and 48 hours. Then, the samples were removed and to each well were added 100 µl of a solution containing 0.5 mg/ml of 3-(4,5-dimethylthiazol-2-yl)-2,5-diphenyltetrazolium bromide (MTT, Sigma-Aldrich). After three hours, the MTT solution was removed and were added 100 µl DMSO (Sigma-Aldrich).

The optical density (OD) was measured with the instrument Synergy HT (BioTek, Winooski, VT, USA) at a wavelength of 570 nm and at a reference wavelength of 670 nm. The cell viability was expressed as percentage (%) of the DOs/DOc ratio, where DOs is the mean value of the optical density of the samples object of the test, and DOc is the average value of untreated cells.

The results are summarized in the following table 2.

**TABLE 2**

| Sample | Dialysis buffer | Glycine (% w/v) | Mannitol (% w/v) | Incubation time (hours) | Concentration (mg/well) | Viability (%) | St. D. |
|---|---|---|---|---|---|---|---|
| 2A1 | TRIZMA | 0.2 | 0 | 24 | 2.5 | 45.1 | 4.48 |
| 2A2 | TRIZMA | 0.2 | 0 | 24 | 1.25 | 57.9 | 3.71 |
| 2A3 | TRIZMA | 0.2 | 0 | 24 | 0.6 | 62.4 | 5.26 |
| 2B1 | TRIZMA | 0.2 | 0 | 48 | 2.5 | 42.3 | 1.93 |
| 2B2 | TRIZMA | 0.2 | 0 | 48 | 1.25 | 59.9 | 10.02 |
| 2B3 | TRIZMA | 0.2 | 0 | 48 | 0.6 | 72.9 | 3.31 |
| 2C1 | NaCl | 0.2 | 0 | 24 | 2.5 | 51.9 | 4.49 |
| 2C2 | NaCl | 0.2 | 0 | 24 | 1.25 | 52.1 | 10.75 |
| 2C3 | NaCl | 0.2 | 0 | 24 | 0.6 | 60.0 | 5.56 |
| 2D1 | NaCl | 0.2 | 0 | 48 | 2.5 | 52.7 | 5.54 |
| 2D2 | NaCl | 0.2 | 0 | 48 | 1.25 | 59.9 | 9.07 |
| 2D3 | NaCl | 0.2 | 0 | 48 | 0.6 | 63.7 | 6.94 |
| 2E1 | NaCl | 0.75 | 0 | 24 | 2.5 | 54.8 | 4.81 |
| 2E2 | NaCl | 0.75 | 0 | 24 | 1.25 | 57.2 | 16.09 |
| 2E3 | NaCl | 0.75 | 0 | 24 | 0.6 | 72.4 | 6.68 |
| 2F1 | NaCl | 0.75 | 0 | 48 | 2.5 | 50.0 | 4.79 |
| 2F2 | NaCl | 0.75 | 0 | 48 | 1.25 | 53.1 | 4.26 |
| 2F3 | NaCl | 0.75 | 0 | 48 | 0.6 | 63.9 | 4.11 |
| 2G1 | NaCl | 0 | 3 | 24 | 2.5 | 73.1 | 1.95 |
| 2G2 | NaCl | 0 | 3 | 24 | 1.25 | 83.2 | 5.34 |
| 2G3 | NaCl | 0 | 3 | 24 | 0.6 | 92.0 | 5.88 |
| 2H1 | NaCl | 0 | 3 | 48 | 2.5 | 66.1 | 7.36 |
| 2H2 | NaCl | 0 | 3 | 48 | 1.25 | 71.9 | 4.98 |
| 2H3 | NaCl | 0 | 3 | 48 | 0.6 | 87.0 | 6.75 |
| 2I1 | NaCl | 0.75 | 3 | 24 | 2.5 | 81.7 | 16.26 |
| 2I2 | NaCl | 0.75 | 3 | 24 | 1.25 | 88.1 | 9.62 |
| 2I3 | NaCl | 0.75 | 3 | 24 | 0.6 | 93.5 | 7.38 |
| 2J1 | NaCl | 0.75 | 3 | 48 | 2.5 | 78.3 | 14.49 |
| 2J2 | NaCl | 0.75 | 3 | 48 | 1.25 | 83.3 | 18.29 |
| 2J3 | NaCl | 0.75 | 3 | 48 | 0.6 | 97.8 | 16.17 |
| 2K1 | TRIZMA | 0.75 | 3 | 24 | 2.5 | 89.9 | 11.10 |
| 2K2 | TRIZMA | 0.75 | 3 | 24 | 1.25 | 93.8 | 4.23 |
| 2K3 | TRIZMA | 0.75 | 3 | 24 | 0.6 | 102.3 | 3.48 |
| 2L1 | TRIZMA | 0.75 | 3 | 48 | 2.5 | 92.6 | 11.19 |
| 2L2 | TRIZMA | 0.75 | 3 | 48 | 1.25 | 96.2 | 3.84 |
| 2L3 | TRIZMA | 0.75 | 3 | 48 | 0.6 | 102.4 | 4.66 |
| 2M1 | PBS | 0.75 | 3 | 24 | 2.5 | 82.6 | 15.16 |
| 2M2 | PBS | 0.75 | 3 | 24 | 1.25 | 85.1 | 11.10 |
| 2M3 | PBS | 0.75 | 3 | 24 | 0.6 | 89.6 | 11.03 |
| 2N1 | PBS | 0.75 | 3 | 48 | 2.5 | 74.7 | 7.15 |
| 2N2 | PBS | 0.75 | 3 | 48 | 1.25 | 85.2 | 13.08 |
| 2N3 | PBS | 0.75 | 3 | 48 | 0.6 | 82.1 | 12.13 |
| 201 | H₂O | 0.75 | 3 | 24 | 2.5 | 93.4 | 14.02 |
| 2O2 | H₂O | 0.75 | 3 | 24 | 1.25 | 93.9 | 3.57 |
| 2O3 | H₂O | 0.75 | 3 | 24 | 0.6 | 97.9 | 5.73 |
| 2P1 | H₂O | 0.75 | 3 | 48 | 2.5 | 91.1 | 13.12 |
| 2P2 | H₂O | 0.75 | 3 | 48 | 1.25 | 104.6 | 15.06 |
| 2P3 | H₂O | 0.75 | 3 | 48 | 0.6 | 98.4 | 16.42 |
| 2Q1 | Na₂HPO₄ | 0.75 | 3 | 48 | 2.5 | 81.1 | 2.81 |
| 2Q2 | Na₂HPO₄ | 0.75 | 3 | 48 | 1.25 | 101.7 | 10.07 |
| 2Q3 | Na₂HPO₄ | 0.75 | 3 | 48 | 0.6 | 95.9 | 4.31 |

The results in table 2 showed that the cell viability is influenced by the type of buffer used, with higher values for the TRIZMA and Na₂HPO₄ buffer and lower values for the NaCl and PBS buffer. Also in this case, the use of water surprisingly showed the best value.

In addition, with the same buffer, using NaCl, mannitol gave better results than glycine, and comparable with those of the glycine-mannitol combination.

### Example 3

For the assessment of integrity of microvesicles and/or exosomes, the samples obtained were subjected to the fluorescence assay with the Nile Red stain.

The lyophilized samples obtained with the procedure of example 1 (with and without dialysis) using the buffer and the cryo-protector of the following table 3 were treated with the Nile Red stain (90 µl of sample at a concentration of 9 mg/ml in PBS and 10 µl of a Nile Red solution) and examined with the Synergy HT plate reader (BioTek, Milan, Italy) at fixed excitation wavelengths and emission (530/25 excitation; 645/40 emission).

**TABLE 3**

| Sample | Dialysis | Dialysis buffer | Glycine (% w/v) | Mannitol (% w/v) | Fluorescence intensity | St. D. |
|---|---|---|---|---|---|---|
| 3A | No | - | 2 | 0 | 16833 | 338.2 |
| 3B | No | - | 0.2 | 0 | 22656 | 1329.4 |
| 3C | No | - | 0.75 | 0 | 14877 | 625.3 |
| 3D | No | - | 0.75 | 3 | 53563 | 42276.9 |
| 3E | No | - | 0 | 3 | 89480 | 1993.7 |
| 3F | Yes | Na₂HPO₄ | 2 | 0 | 26819 | 5957.1 |
| 3G | Yes | Na₂HPO₄ | 2 | 0 | 43647 | 2406.7 |
| 3H | Yes | TRIZMA | 0.2 | 0 | 56541 | 22478.4 |
| 3I | Yes | NaCl | 0.2 | 0 | 76508 | 19940.5 |
| 3J | Yes | NaCl | 0.75 | 0 | 91696 | 617.2 |
| 3K | Yes | NaCl | 0 | 3 | 94419 | 1140.6 |
| 3L | Yes | NaCl | 0.75 | 3 | 95297 | 383.0 |
| 3M | Yes | TRIZMA | 0.75 | 3 | 67404 | 4914.8 |
| 3N | Yes | PBS | 0.75 | 3 | 93373 | 11637.5 |
| 3O | Yes | H₂O | 0.75 | 3 | 57366 | 39860.8 |
| 3P | Yes | Na₂HPO₄ | 0.75 | 3 | 80851 | 7206.6 |

The results in table 3 showed a greater fluorescence intensity for the samples subjected to dialysis, demonstrating a greater integrity of microvesicles and/or exosomes.

In addition, the fluorescence intensity was influenced in a substantial manner, with the same cryo-protector, by the type of buffer used. The best results were obtained with the NaCl and PBS buffer, while in this case the use of water provided the less satisfactory results.

On the contrary, with the same buffer, no significant differences were observed in the use of mannitol or glycine or mixtures thereof by using NaCl.

Finally, samples obtained with culture in serum-free medium for 24 hours produced a greater fluorescence intensity, and hence greater integrity of microvesicles and/or exosomes, with respect to samples obtained with culture in serum-free medium for 4 hours only.

### Example 4

The samples obtained according to example 2 were analyzed to determine the dimensions thereof by light scattering, using a DelsaMax CORE analyzer (Beckman Coulter, Krefeld, Germany), with a scanning electron microscope (SEM), the JEOL JEM 1200EX transmission electron microscope (TEM) (Jeol, Tokyo, Japan), and the TCS SP5 II confocal laser scanning (Leica Microsystems, Wetzlar, Germany).

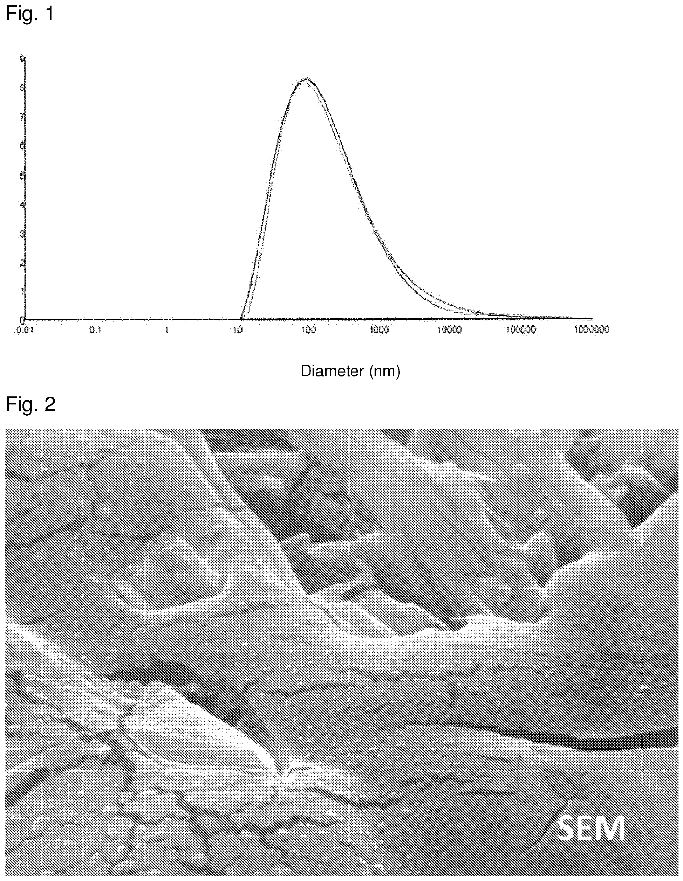

Figure 1 shows the size distribution graph of microvesicles obtained with the light scattering technique. The average particle size was found to be 115 nm with a polydispersity index (PDI) equal to 0.255.

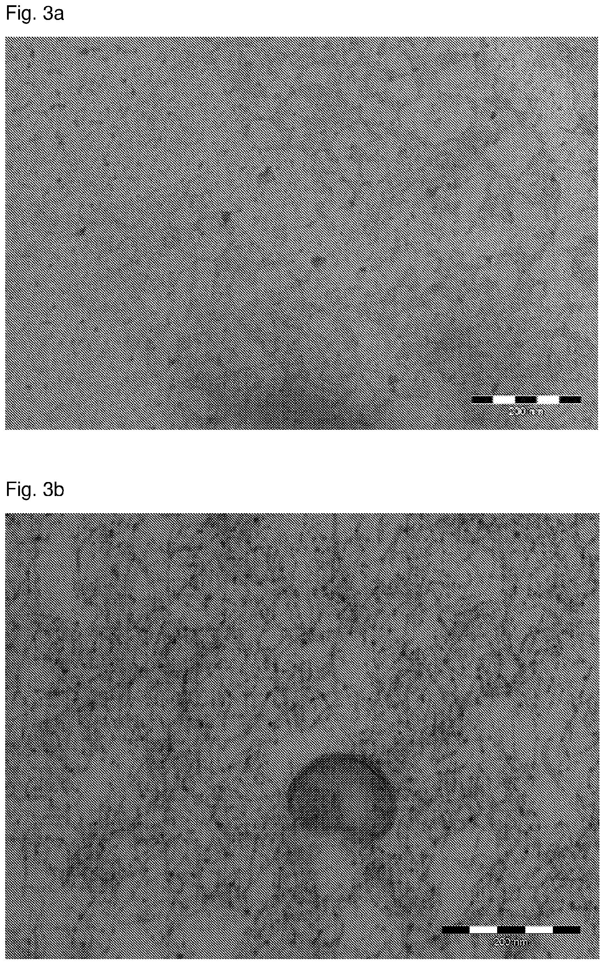

The morphological characterization performed with a scanning electron microscope (SEM) and transmission electron microscope (TEM) confirmed that the microvesicles retained their spherical structure with a smooth surface, as shown in Figure 2 (SEM) and in figures 3a and 3b (TEM).

The images obtained with the confocal laser scanning microscope on samples treated with Nile Red stain confirmed the integrity of the microvesicles and/or exosomes showing a well-defined fluorescence.

### Example 5

Finally, the samples obtained were evaluated for their ability to inhibit the proliferation of human peripheral blood mononuclear cells induced by phytohemagglutinin (PHA) and to inhibit inflammation in a model of inflammation induced by IL-1 βon human chondrocytes.

### Immunomodulation assay

Human peripheral blood mononuclear cells (PBMC) were isolated from heparinized venous blood of healthy volunteers by centrifugation in a density gradient (Ficoll-Paque Plus). The PBMCs were counted and placed in a 96-well plate (Corning Costar, Celbio) in an amount of 100,000 cells per well, in RPMI 1640 medium (Sigma-Aldrich) supplemented with 10% fetal bovine serum (FBS) (Euroclone) with or without sample of microvesicles and/or exosomes obtained by following the procedure of example 1, using water as the dialysis buffer and glycine/mannitol as a cryo-protector (0-30 mg/ml) and/or phytohemagglutinin (PHA, 10 µg/ml) in duplicate.

After 72 hours, the samples were collected and the proliferation, cell viability and the production of IL-6 thereof was assessed.

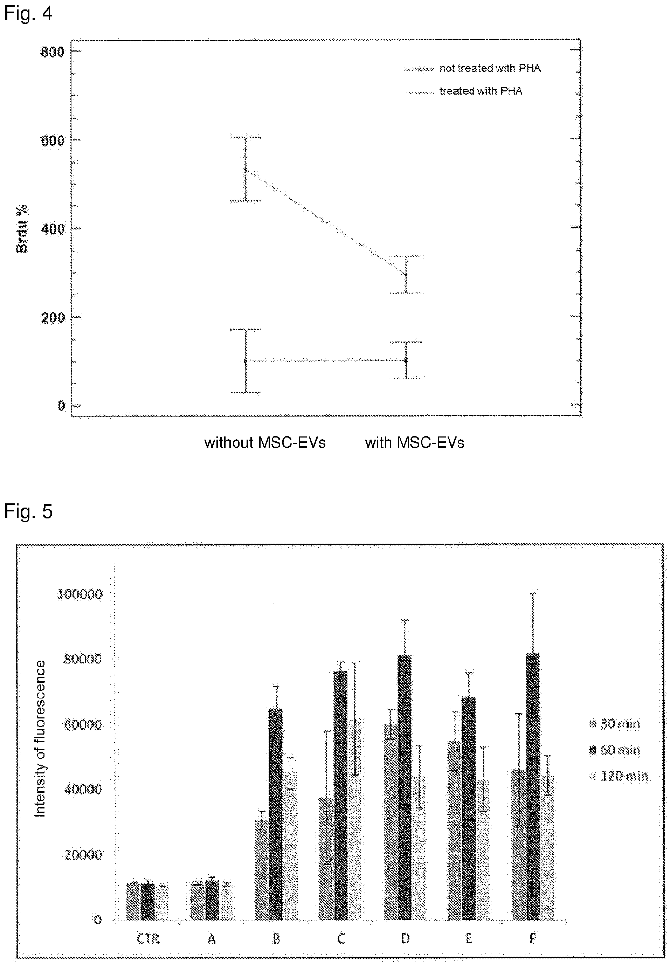

The proliferation was assessed with the proliferation assay with bromodeoxyuridine (ELISA BrdU - Life Science Roche) following the manufacturer's instructions. The results are shown in Figure 4, which shows a significant inhibitory effect of the proliferation of PBMC by the sample containing dialyzed and lyophilized microvesicles and/or exosomes.

The cell viability was assessed by the addition of the resazurin dye (Alamar Blue) and measurement of fluorescence, after 4 hours of incubation at 37°C, with a spectrophotometer (Victor X3, Perkin Elmer) with excitation wavelength equal to 540 nm and emission wavelength equal to 590 nm.

The results confirmed that the PBMC exposure to different doses of lyophilized microvesicles and/or exosomes had no toxic effects on the cells, retaining the viability value above 90% in all samples analyzed.

The production of IL-6 was assessed using ELISA (Mabtech, 2 pg/ml detection limit, 5% intra-assay variation) in PBMC samples activated with phytohemagglutinin (PHA) in the presence and in the absence of lyophilized microvesicles and/or exosomes.

The activation with PHA resulted in a significant increase in the concentration of IL-6 compared to the control, reaching values greater than 3000 pg/ml. Following the addition to the culture medium of lyophilized microvesicles and/or exosomes, this value was significantly reduced, bringing the concentration of IL-6 around 1740 pg/ml (-58%).

### IL-1 assayβ on chondrocyte cultures

Human articular chondrocyte cells (ACC) were isolated from waste cartilage fragments resulting from total hip arthroplasty by enzymatic digestion with type II collagenase. The ACC were cultured by a few passages in culture prior to treatment with the pro-inflammatory cytokine IL-1β, in the absence or presence of microvesicles and/or exosomes obtained by following the procedure of example 1, using water as the dialysis buffer and glycine/mannitol as a cryo-protector (0-30 mg/mL).

Cells and supernatants were collected after 48 hours of treatment. The gene expression was assessed by RT-PCR and the production of pro- and anti-inflammatory catabolic markers was analyzed by ELISA assay.

The results showed a strong inflammation inhibitory activity, observing a down-regulation of MMP-1, VEGF and TNFα and an up-regulation of TMP-1 TGFβ and IL-1Ra.

### Example 6

The procedure described in example 1 to isolate the mesenchymal stem cells from adipose tissue was repeated.

The MSC thus obtained were seeded in a 96-well plate in a single layer with a density of 10,000 cells/cm², and incubated in the DMEM/F12 culture medium (1:1 ratio) supplemented with 10% fetal bovine serum (FBS) for 24 hours at 37°C in a 5% CO₂ atmosphere.

Then, to each well were added 200 µg/ml of nanoparticles loaded with curcumin according to the following table 4, prepared with the desolvation in acetone method (Seib FP et al., "pH-Dependent anticancer drug release from silk nanoparticles", Adv Healthc Mater. 2013 December; 2/12), continuing the incubation for 30, 60 or 120 minutes.

**TABLE 4**

| Nanoparticles | Concentration of curcumin in acetone (mg/ml) | Loaded curcumin (%) | St. D. |
|---|---|---|---|
| Np0 | 0 | 0 | - |
| Np1 | 0.03 | 0.95 | 0.127 |
| Np2 | 0.1 | 1.50 | 0.111 |
| Np3 | 0.5 | 13.66 | 1.518 |
| Np4 | 1.5 | 31.97 | 1.522 |

At the predetermined times, the MSC were washed with PBS and the cellular uptake of the nanoparticles was evaluated in terms of fluorescence intensity, exploiting the fluorescent properties of curcumin, measured with a microplate reader (Synergy HT, BioTek, United Kingdom) equipped with an excitation filter at 485 nm and an emission filter at 528 nm. The results are shown in Figure 2.

The results obtained for the same incubation times demonstrated the direct proportionality between fluorescence intensity and amount of curcumin present in the nanoparticles, with increasing values passing from Np1 to Np4, while fluorescence was minimal in untreated MSC or MSC treated with Np0.

The results obtained with the same nanoparticles showed that the maximum uptake was obtained after 60 minutes. In fact, at 30 minutes the fluorescence intensity was lower than that obtainable at 60 minutes, while at 120 minutes no further significant increase in the fluorescence intensity was obtained.

The MSC loaded with nanoparticles Np3 and incubated for 60 minutes were cultured with minimal medium overnight, and then the supernatant was separated to assess the presence of microvesicles and/or exosomes loaded with nanoparticles, again exploiting the fluorescent properties of curcumin.

The results confirmed the presence of nanoparticles Np3 in the microvesicles and/or exosomes released into the supernatant by the MSC.

### Example 7

In order to test the effect of the cryo-protector on the quality and stability of the product after lyophilization, four samples were prepared at different concentrations of mannitol (0.0%, 0.5%, 1.5% and 3% w/v). The samples were prepared as follows.

Mesenchymal stem cells (MSC) were isolated from human adipose tissue as described in example 1.

Upon reaching sub-confluence, the MSC were transferred and cultured in DMEM/F12 minimal medium for 9 and 24 hours. After 9 hours, the supernatant was removed and replaced with fresh DMEM/F12 minimal medium. After 24 hours from the beginning of the process, the supernatant was collected and added to that collected previously. The MSC were removed by treatment with 0.05% trypsin-EDTA (Euroclone, Milan, Italy) to assess the number of cells and their viability. The supernatant was ultrafiltered with the tangential ultrafiltration method with a filtering module having cut-off of 5000 Dalton (Spectrum Laboratories, Milan, Italy).

Water was used as dialysis medium. The ultrafiltered product was divided into four aliquots: the first aliquot was used as such (and therefore devoid of cryo-protector) while the other three aliquots were admixed with increasing concentrations of cryo-protector (0.5%, 1.5% and 3% w/v mannitol). All samples were frozen at -20°C, and then subjected to the lyophilization process with vacuum below 1.33 mbar.

The samples obtained (7A-7D) were subjected to the BCA assay with the Protein Assay Kit from Thermo Scientific (Milan, Italy) for the quantification of the proteins contained in the lyophilized products.

The total protein content of the lyophilized samples of microvesicles and/or exosomes was determined using the BCA-Protein Assay kit (Thermo Scientific, Milan, Italy), according to the manufacturer's specifications.

The absorbance-concentration calibration curve was generated using standard bovine serum albumin (BSA, Sigma-Aldrich). The working reagent solution was added to each sample or standard (1:1 ratio), and the sets were then incubated at 37°C for 2 hours before measuring the absorbance at 562 nm with a microplate reader (Synergy HT, BioTek, United Kingdom). The protein concentration was extrapolated from a concentration graph against absorbance obtained from standard protein solutions, using a third degree polynomial equation, with r² 0.99 for each assay.

The results were processed using the two-way analysis of variance, considering the concentration of mannitol and the lyophilization process (yes/no) as fixed factors. Significance was set at 0.05.

The results are summarized in the following table 5.

**TABLE 5**

| Sample | Incubation in minimal medium (hours) | Purification method | Dialysis buffer | Mannitol (% w/v) | Pre-lyo | | Post-lyo | |
|---|---|---|---|---|---|---|---|---|
| | | | | | Proteins (µg/ml) | St. D. | Proteins (µg/ml) | St. D. |
| 7A | 9+24 | Ultrafiltration | H₂O | 0 | 158.14 | 7.935 | 135.0 | 5,413 |
| 7B | 9+24 | Ultrafiltration | H₂O | 0.5 | 174.59 | 0.750 | 175.2 | 15.26 |
| 7C | 9+24 | Ultrafiltration | H₂O | 1.5 | 177.62 | 7.385 | 176.7 | 13.010 |
| 7D | 9+24 | Ultrafiltration | H₂O | 3 | 190.17 | 11.712 | 172.5 | 5.413 |

The lyophilized product (7A) without cryo-protector appeared as a light yellow uniform mass, non-friable, of gluey consistency, strongly adherent to the walls of the container, not easily removable and manipulable, with very poor flow and flowability properties (not quantifiable).

The lyophilized product (7B-C) with the addition of cryo-protector (even at the lowest concentration) appears as a white powder of homogeneous appearance, porous and friable, non-adherent to the walls of the container, easily removable and manipulable, with good flow and flowability properties.

The results of the quantitative analysis of the protein content shown in table 5 showed that the absence of cryo-protector leads to a significant reduction in the protein content after the lyophilization process, while the addition of cryo-protector (even in a minimum concentration) does not lead to a significant reduction in the protein content as a result of the lyophilization process.

### Example 8

The sample preparation procedure described in example 7 was repeated using both the ultrafiltration method and the dialysis method with dialysis membranes having a cut-off of 3500 Dalton (Spectra/Por, Spectrum Laboratories, Milan, Italy) and using as glycine and/or mannitol cryo-protector in the amounts indicated in the following table 7, which also illustrates the percentage composition of the samples (8A-8T) obtained after lyophilization.

**TABLE 7**

| **Sample** | **Isolation method** | **Percentage composition of the solution (% w/v)** | | **Percentage composition of the lyophilized product (% w/w)** | | |
|---|---|---|---|---|---|---|
| | | **Glycine** | **Mannitol** | **Glycine** | **Mannitol** | **Secretome** |
| 8A | Dialysis | 0.75 | 3 | 11.76 | 47.02 | 41.22 |
| 8B | Dialysis | 0.75 | 3 | 10.53 | 42.13 | 47.33 |
| 8C | Dialysis | 0.75 | 3 | 10.43 | 41.71 | 47.86 |
| 8D | Dialysis | 0.75 | 3 | 11.50 | 45.99 | 42.51 |
| 8E | Dialysis | 0.75 | 3 | 11.19 | 44.78 | 44.03 |
| 8F | Dialysis | 0.75 | 3 | 10.34 | 41.35 | 48.31 |
| 8G | Dialysis | 0 | 3 | 0 | 55.65 | 44.35 |
| 8H | Dialysis | 0 | 3 | 0 | 58.25 | 41.75 |
| 8I | Dialysis | 0 | 3 | 0 | 61.48 | 38.52 |
| 8L | Dialysis | 1.75 | 3 | 26.76 | 45.87 | 27.37 |
| 8M | Dialysis | 1.75 | 3 | 25.84 | 44.30 | 29.87 |
| 8N | Dialysis | 1.75 | 3 | 24.07 | 41.27 | 34.66 |
| 80 | Dialysis | 2 | 3 | 26.27 | 39.41 | 34.31 |
| 8P | Dialysis | 2 | 3 | 29.03 | 43.54 | 27.43 |
| 8Q | Dialysis | 2 | 3 | 22.12 | 33.19 | 44.69 |
| 8R | Ultrafiltration | 0.25 | 0.5 | 28.47 | 56.93 | 14.60 |
| 8S | Ultrafiltration | 0 | 0.5 | 0 | 81.08 | 18.92 |
| 8T | Ultrafiltration | 0 | 1.5 | 0 | 94.94 | 5.06 |

The percentage composition of the lyophilized product has a secretome content ranging between about 5% and about 48% w/w. The isolation method influences the percentage composition of the lyophilized product, with higher percentages of secretome for samples obtained after dialysis compared to ultrafiltration.

### Example 9

Membranes made from sodium alginate comprising the lyophilized powder obtained by the process of the present invention were used in an animal wound model to assess the efficacy thereof in the regeneration and healing process.

### Preparation of the membranes

The low viscosity sodium alginate powder (Sigma-Aldrich, Milan, Italy) was dissolved in distilled water to a final concentration of 1% w/v by magnetic stirring. To the solution thus obtained was added the secretome obtained according to dialysis and/or ultrafiltration, as described in example 1, using ultrapure deionized water as dialysis/ultrafiltration fluid, prior to its lyophilization, at a final concentration of 2x10⁶ equivalent cells per membrane. 500 ml of the final mixture were transferred to each well of a 24 Multiwell (0.5 ml/1 cm² surface) and the preparations were lyophilized at 8*10⁻¹ mbar and -50°C for 72 h (Modulyo^{®} Edwards Freeze dryer, Kingston, NY). The alginate-only membranes (1% w/v) were prepared in a similar manner and used as a control. According to another procedure, to the solution of 1% w/v alginate was added the secretome in powder, obtained as described in example 1, by dialysis and/or ultrafiltration, addition of the cryo-protector (0.5% w/v mannitol) and subsequent lyophilization, at a final concentration of 2x10⁶ equivalent cells per membrane.

### Animal wound model

All animal experiments were performed according to the international NIH Guide for the Care and Use of Laboratory Animals and institutional guidelines, after the ICC Ethics Committee approval for animal testing (CSEA) of IRCCS AOU San Martino-IST. Nine male C57BL mice eight to 12 weeks old (Charles River, MA, USA) were used for the ulcerative generation model. The surgical procedures were performed following intraperitoneal anesthesia induced with xylazine/ketamine. During anesthesia, the hair from the dorsal surface of each animal was removed with a depilatory cream and the bare skin was cleaned with a betadine solution. A 6mm sterile biopsy punch was used to create a wound through the whole thickness, extending through the panniculus carnosus. The membranes produced according to the different compositions and procedures described above were applied to cover the entire area of the wound. Later, the lesions were covered with a non-adherent dressing (Tegaderm). Digital images were acquired to document the size of the wound during the treatment follow-up. At different times, after the mice were euthanized, the treated skin lesion region was collected and prepared for histological analysis.

The samples collected at predetermined post-surgical times (3, 7 and 21 days) were fixated with 4% paraformaldehyde at room temperature for 24 hours before the addition of paraffin to obtain tissue sections. The 5 um thick sections were stained with the use of hematoxylin-eosin and Masson Trichrome. For each sample, images of different areas were acquired by a Zeiss Axiovert 200M microscope (Zeiss, Milan, Italy) at x100 magnification. The regenerated area evaluation was carried out by using parameters for the evaluation of the wound healing process, as described in the literature (Abramov et al., "Histologic characterization of vaginal vs. abdominal surgical wound healing in a rabbit model", Wound Rep Reg (2007) 15 80-86).

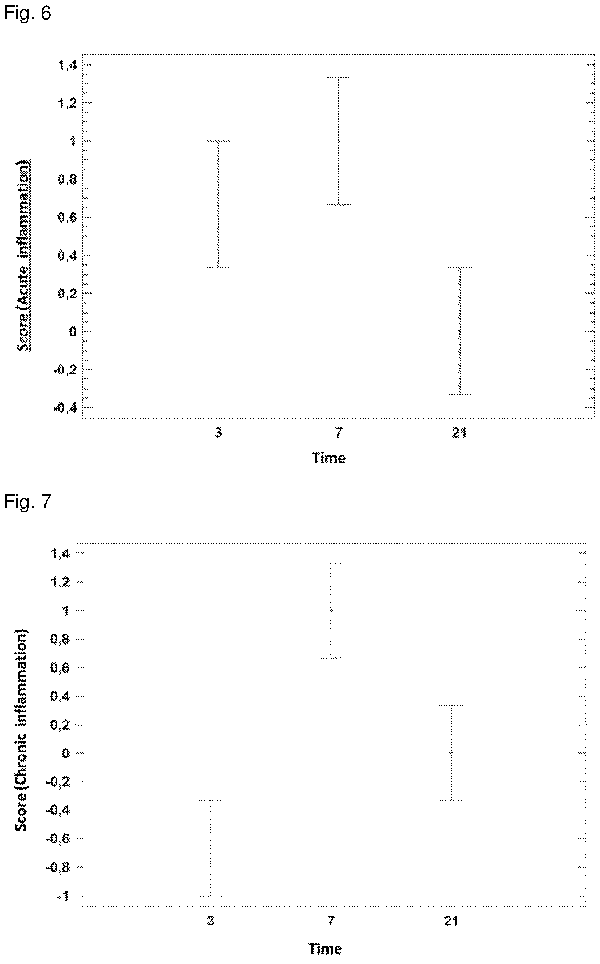

The scores taken into account were related to acute and chronic inflammation and to the deposition of granulation tissue, according to Abramov parameters (2007). In order to eliminate the variability among animals, for each mouse was calculated the difference between the score assigned to the treated lesion (with secretome) and that attributed to the control lesion (alginate alone). The scores obtained were treated by ANOVA multifactor. The results are shown in the following Table 8 and in Figures 6-8.

**TABLE 8**

| **Time (days)** | **Score according to Abramov** | | | | | |
|---|---|---|---|---|---|---|
| | **Acute inflammation** | **Chronic inflammation** | **Deposition of granulation tissue** | **Scoring average ± standard deviation** | | |
| | | | | **Acute inflammation** | **Chronic inflammation** | **Deposition of granulation tissue** |
| 3 | 1 | -1 | 0 | 0.66 ± 0.577 | -0.66 ± 0.577 | 0 ± 0 |
| | 1 | -1 | 0 | | | |
| | 0 | 0 | 0 | | | |
| 7 | 1 | 1 | 0 | 1 ± 0 | 1 ± 0 | 0 ± 0 |
| | 1 | 1 | 0 | | | |
| | 1 | 1 | 0 | | | |
| 21 | 0 | 0 | 1 | 0 ± 0 | 0 ± 0 | 1 ± 0 |
| | 0 | 0 | 1 | | | |
| | 0 | 0 | 1 | | | |

The results suggest greater effectiveness of the alginate/secretome membranes with respect to alginate only membranes in the wound healing process. No differences were found between the use of fresh secretome and lyophilized secretome.

Acute inflammation is an essential defense mechanism put in place by the body to eliminate cell debris formed as a result of tissue damage, thus triggering the healing process. However, the prolongation of an acute inflammation can be harmful. Treatment with secretome induced an acute inflammatory process which at 3 and 7 days was prevalent on the controls and which decreased gradually up to 21 days, when there were no significant differences between treated and control (Fig. 6).

Acute inflammation extinction is essential to prevent the onset of a chronic inflammatory process that leads to a delay in wound healing and to scar formation. Treatment with secretome delayed the onset of a chronic inflammatory process in the short term (at 3 days it prevailed in the controls with respect to the treated ones), while at 21 days there was no significant difference between treated and control (Fig. 7).

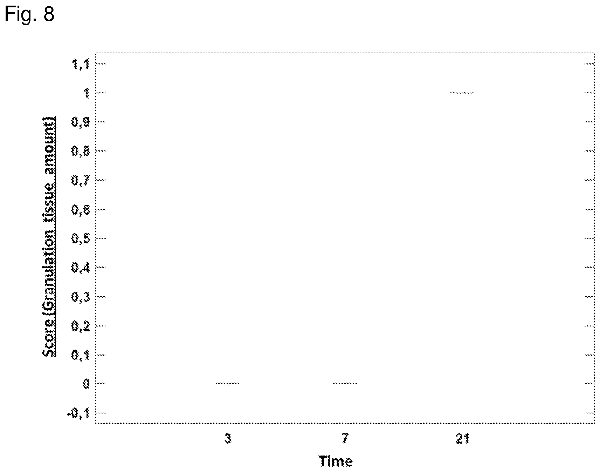

Finally, treatment with secretome led to an increased deposition of granulation tissue at 21 days with respect to the control, showing the onset of the neo-angiogenesis, which is a process of formation of new blood vessels that occurs as a result of the massive production of VEGF (Fig. 8).

## Claims

1. A production process of microvesicles and/or exosomes, **characterized in that** it comprises the following steps:
(i) collecting a biological fluid comprising microvesicles and/or exosomes,
(ii) dialyzing or ultrafiltering said biological fluid using a membrane having a threshold value (cut-off) equal to or smaller than 5,000 Dalton and a dialysis or ultrafiltration fluid,
(iii) adding a cryo-protector to the dialyzed or ultrafiltered solution obtained from step (ii), and
(iv) lyophilizing the solution resulting from step (iii) obtaining a lyophilized powder comprising microvesicles and/or exosomes in an amount equal to or greater than 20% w/w with respect to the total weight of said lyophilized powder; and at least one cryo-protector in an amount equal to or smaller than 80% w/w.

2. The production process of microvesicles and/or exosomes according to claim 1, **characterized in that** said biological fluid is a supernatant of cultured cells, preferably collected from cells cultured in a culture medium, in particular a minimal culture medium, for at least 4 hours.

3. The production process of microvesicles and/or exosomes according to claim 2, **characterized in that** said cells are stem cells and specifically mesenchymal stem cells.

4. The production process of microvesicles and/or exosomes according to claim 3, **characterized in that** said stem cells are mesenchymal stem cells isolated from adipose tissue by washing with buffer solution and enzymatic digestion.

5. The production process of microvesicles and/or exosomes according to any one of the preceding claims 2 to 4, **characterized in that** said cells are cultured in a culture medium containing nanoparticles loaded with a biologically active substance.

6. The production process of microvesicles and/or exosomes according to claim 1, **characterized in that** said biological fluid is a bodily fluid.

7. The production process of microvesicles and/or exosomes according to claim 1, **characterized in that** said dialysis or ultrafiltration fluid is water or a buffer solution with a concentration of salts smaller than the concentration of salts of the biological fluid.

8. The production process of microvesicles and/or exosomes according to claim 1, **characterized in that** said dialysis fluid comprises a polyoxyalkylene having an average molecular weight equal to or greater than 100 g/mol, for example a polyethylene glycol (PEG) having an average molecular weight equal to or greater than 100 g/mol, preferably equal to or greater than 300 g/mol, more preferably equal to or greater than 500 g/mol, and equal to or smaller than 4000 g/mol, preferably equal to or smaller than 3000 g/mol, more preferably equal to or smaller than 2500 g/mol.

9. The production process of microvesicles and/or exosomes according to claim 1, **characterized in that** said cryo-protector is selected from the group consisting of sugars, amino acids, polyols or mixtures thereof.

10. The production process of microvesicles and/or exosomes according to claim 9, **characterized in that** said cryo-protector is selected from the group consisting of mannitol, lactose, sucrose, trehalose, sorbitol, glucose, maltose, fructose, raffinose, arginine, alanine, glycine, histidine, ethylene glycol, 1,3-propanediol, glycerol, cyclo-dextrins and mixtures thereof.

11. The production process of microvesicles and/or exosomes according to claim 10, **characterized in that** said cryo-protector is selected from the group consisting of mannitol, glycine or mixtures thereof.

12. The production process of microvesicles and/or exosomes according to claim 1, **characterized in that** the lyophilization step (iv) comprises the following steps:
a) freezing the dialyzed or ultrafiltered solution obtained from step (iii) operating at a temperature lower than -50°C, preferably lower than -70°C,
b) sublimating the frozen solution under vacuum in a lyophilizer.

13. Product in the form of lyophilized powder obtained by the process according to any one of the preceding claims, comprising microvesicles and/or exosomes having a diameter between 20 and 5,000 nm and an average diameter between 50 and 200 nm, and at least one cryo-protector; wherein said lyophilized powder comprises said microvesicles and/or exosomes in an amount equal to or greater than 20% w/w with respect to the total weight of said lyophilized powder; and said at least one cryo-protector in an amount equal to or smaller than 80% w/w.

14. Product according to claim 13, **characterized in that** said cryo-protector is selected from the group consisting of sugars, amino acids, polyols or mixtures thereof.

15. Product according to any one of claims 13 or 14, **characterized in that** said lyophilized powder comprises said microvesicles and/or exosomes in an amount equal to or greater than 30% w/w and preferably equal to or greater than 40% w/w with respect to the total weight of said lyophilized powder; and said at least one cryo-protector in an amount equal to or smaller than 70% w/w, preferably equal to or smaller than 60% w/w with respect to the total weight of said lyophilized powder.

16. Product according to any one of claims 13 or 14, **characterized in that** said lyophilized powder contains said microvesicles and/or exosomes in an amount comprised between 20% and 60% w/w, and said at least one cryo-protector in an amount comprised between 80% and 40% w/w.

17. Product according to claim 13, **characterized in that** said microvesicles and/or exosomes comprise nanoparticles loaded with a biologically active substance.

18. Product according to claim 17, **characterized in that** said biologically active substance is selected from the group consisting of cardiovascular drugs, drugs of the central and peripheral nervous system, painkilling drugs, antimicrobial drugs, chemotherapy drugs, anti-tumor drugs, anti-tumor immunotherapy drugs, vaccines, hormones, peptide and protein drugs, vitamins, plant extracts and phytocomplexes.

19. Pharmaceutical composition comprising a biologically active substance and at least one pharmaceutically acceptable excipient, said biologically active substance being loaded into nanoparticles comprised in lyophilized microvesicles and/or exosomes having a diameter between 20 and 5,000 nm and an average diameter between 50 and 200 nm; **characterized in that** said lyophilized microvesicles and/or exosomes are comprised in a product in the form of lyophilized powder according to any one of claims 13 to 18 together with at least one cryo-protector.

20. Pharmaceutical composition according to claim 19, **characterized in that** said biologically active substance is selected from the group consisting of cardiovascular drugs, drugs of the central and peripheral nervous system, painkilling drugs, antimicrobial drugs, chemotherapy drugs, anti-tumor drugs, anti-tumor immunotherapy drugs, vaccines, hormones, peptide and protein drugs, vitamins, plant extracts and phytocomplexes.

21. Pharmaceutical composition according to claim 20, **characterized in that** said cardiovascular drugs are selected from the group consisting of antihypertensives, antiarrhythmic drugs and diuretics.

22. Pharmaceutical composition according to claim 20, **characterized in that** said drugs of the central and peripheral nervous system are selected from the group consisting of anesthetics, hypnotics, sedatives, anxiolytics and anti-Parkinson's drugs.

23. Pharmaceutical composition according to claim 20, **characterized in that** said painkilling drugs are selected from the group consisting of anti-inflammatory and analgesic drugs.

24. Pharmaceutical composition according to claim 20, **characterized in that** said antimicrobial drugs are selected from the group consisting of sulfamidic drugs, antibiotics, antivirals and antifungal drugs.

25. Pharmaceutical composition according to any one of the preceding claims 19 to 24 for use in the treatment of arthrosis, cardiovascular diseases, respiratory diseases, neurological diseases, pain, bone marrow failure, diabetes and cancer.

26. Cosmetic or cosmeceutic composition comprising a biologically active substance and at least one cosmetically acceptable excipient, said biologically active substance being loaded into nanoparticles comprised in lyophilized microvesicles and/or exosomes having a diameter between 20 and 5,000 nm and an average diameter between 50 and 200 nm; **characterized in that** said lyophilized microvesicles and/or exosomes are comprised in a lyophilized powder according to any one of claims 13 to 18 together with at least one cryo-protector.

27. Cosmetic or cosmeceutic composition according to claim 26 for use in skin anti-aging treatment.

28. Pharmaceutical composition comprising microvesicles and/or exosomes having a diameter between 20 and 5,000 nm and an average diameter between 50 and 200 nm and at least one pharmaceutically acceptable excipient for the topical treatment of injuries and acute and chronic wounds; **characterized in that** said lyophilized microvesicles and/or exosomes are comprised in a lyophilized powder according to any one of claims 13 to 18 together with at least one cryo-protector.

29. Pharmaceutical composition according to claim 28, wherein said pharmaceutically acceptable excipient comprises a membrane for advanced dressings, and said membrane for advanced optionally comprises sodium alginate, calcium alginate, cellulose derivatives (preferably carboxymethylcellulose), esters of hyaluronic acid, silk proteins and non-woven fabric.

## Patentansprüche

1. Verfahren zur Herstellung von Mikrovesikeln und/oder Exosomen, **dadurch gekennzeichnet, dass** es die folgenden Schritte umfasst:
(i) Sammeln einer biologischen Flüssigkeit, die Mikrovesikel und/oder Exosomen umfasst,
(ii) Dialysieren oder Ultrafiltrieren der biologischen Flüssigkeit unter Verwendung einer Membran mit einem Schwellenwert (Cut-Off) gleich oder kleiner als 5.000 Dalton und einer Dialyse- oder Ultrafiltrationsflüssigkeit,
(iii) Zugabe eines Kryo-Schutzmittels zu der in Schritt (ii) erhaltenen dialysierten oder ultrafiltrierten Lösung, und
(iv) Lyophilisieren der in Schritt (iii) erhaltenen Lösung, wobei ein lyophilisiertes Pulver erhalten wird, das Mikrovesikel und/oder Exosomen in einer Menge von 20 Gew.-% oder mehr, bezogen auf das Gesamtgewicht des lyophilisierten Pulvers, und mindestens ein Kryo-Schutzmittel in einer Menge von 80 Gew.-% oder weniger aufweist.

2. Verfahren zur Herstellung von Mikrovesikeln und/oder Exosomen nach Anspruch 1, **dadurch gekennzeichnet, dass** die biologische Flüssigkeit ein Überstand von kultivierten Zellen ist, vorzugsweise von Zellen, die in einem Kulturmedium, insbesondere einem Minimal-Kulturmedium, während mindestens 4 Stunden kultiviert wurden.

3. Verfahren zur Herstellung von Mikrovesikeln und/oder Exosomen nach Anspruch 2, **dadurch gekennzeichnet, dass** die Zellen Stammzellen und insbesondere mesenchymale Stammzellen sind.

4. Verfahren zur Herstellung von Mikrovesikeln und/oder Exosomen nach Anspruch 3, **dadurch gekennzeichnet, dass** es sich bei den Stammzellen um mesenchymale Stammzellen handelt, die aus Fettgewebe durch Waschen mit Pufferlösung und enzymatischen Aufschluss isoliert wurden.

5. Verfahren zur Herstellung von Mikrovesikeln und/oder Exosomen nach einem der vorhergehenden Ansprüche 2 bis 4, **dadurch gekennzeichnet, dass** die Zellen in einem Kulturmedium kultiviert werden, das mit einer biologisch aktiven Substanz beladene Nanopartikel enthält.

6. Verfahren zur Herstellung von Mikrovesikeln und/oder Exosomen nach Anspruch 1, **dadurch gekennzeichnet, dass** die biologische Flüssigkeit eine Körperflüssigkeit ist.

7. Verfahren zur Herstellung von Mikrovesikeln und/oder Exosomen nach Anspruch 1, **dadurch gekennzeichnet, dass** die Dialyse- oder Ultrafiltrationsflüssigkeit Wasser oder eine Pufferlösung mit einer geringeren Salzkonzentration als die Salzkonzentration der biologischen Flüssigkeit ist.

8. Verfahren zur Herstellung von Mikrovesikeln und/oder Exosomen nach Anspruch 1, **dadurch gekennzeichnet, dass** die Dialyseflüssigkeit ein Polyoxyalkylen mit einem mittleren Molekulargewicht von 100 g/mol oder mehr umfasst, beispielsweise ein Polyethylenglykol (PEG) mit einem mittleren Molekulargewicht von 100 g/mol oder mehr, vorzugsweise von 300 g/mol oder mehr, besonders bevorzugt von 500 g/mol oder mehr, und von 4000 g/mol oder weniger, vorzugsweise von 3000 g/mol oder weniger, besonders bevorzugt von 2500 g/mol oder weniger.

9. Verfahren zur Herstellung von Mikrovesikeln und/oder Exosomen nach Anspruch 1, **dadurch gekennzeichnet, dass** das Kryo-Schutzmittel ausgewählt ist aus der Gruppe bestehend aus Zuckern, Aminosäuren, Polyolen oder deren Mischungen.

10. Verfahren zur Herstellung von Mikrovesikeln und/oder Exosomen nach Anspruch 9, **dadurch gekennzeichnet, dass** das Kryo-Schutzmittel ausgewählt ist aus der Gruppe bestehend aus Mannitol, Lactose, Saccharose, Trehalose, Sorbitol, Glucose, Maltose, Fructose, Raffinose, Arginin, Alanin, Glycin, Histidin, Ethylenglycol, 1,3-Propandiol, Glycerin, Cyclo-Dextrinen und Mischungen davon.

11. Verfahren zur Herstellung von Mikrovesikeln und/oder Exosomen nach Anspruch 10, **dadurch gekennzeichnet, dass** das Kryo-Schutzmittel ausgewählt ist aus der Gruppe bestehend aus Mannitol, Glycin oder deren Mischungen.

12. Verfahren zur Herstellung von Mikrovesikeln und/oder Exosomen nach Anspruch 1, **dadurch gekennzeichnet, dass** der Lyophilisierungsschritt (iv) die folgenden Schritte umfasst:
a) Einfrieren der dialysierten oder ultrafiltrierten Lösung, die in Schritt (iii) erhalten wurde, bei einer Temperatur von weniger als -50°C, vorzugsweise weniger als als -70°C,
b) Sublimation der gefrorenen Lösung unter Vakuum in einem Gefriertrockner.

13. Produkt in Form eines gefriergetrockneten Pulvers, erhalten durch das Verfahren nach einem der vorhergehenden Ansprüche, umfassend Mikrovesikel und/oder Exosomen mit einem Durchmesser zwischen 20 und 5000 nm und einem mittleren Durchmesser zwischen 50 und 200 nm und mindestens ein Kryo-Schutzmittel; wobei das gefriergetrocknete Pulver die Mikrovesikel und/oder Exosomen in einer Menge gleich oder größer als 20 Gew.-%, bezogen auf das Gesamtgewicht des gefriergetrockneten Pulvers, umfasst; und das mindestens eine Kryo-Schutzmittel in einer Menge gleich oder kleiner als 80 Gew.-%.

14. Produkt nach Anspruch 13, **dadurch gekennzeichnet, dass** der Kryoprotektor ausgewählt ist aus der Gruppe bestehend aus Zuckern, Aminosäuren, Polyolen oder Mischungen davon.

15. Produkt nach einem der Ansprüche 13 oder 14, **dadurch gekennzeichnet, dass** das gefriergetrocknete Pulver die Mikrovesikel und/oder Exosomen in einer Menge gleich oder größer als 30 Gew.-% und vorzugsweise gleich oder größer als 40 Gew.-%, bezogen auf das Gesamtgewicht des gefriergetrockneten Pulvers, und das mindestens eine Kryo-Schutzmittel in einer Menge gleich oder kleiner als 70 Gew.-%, vorzugsweise gleich oder kleiner als 60 Gew.-%, bezogen auf das Gesamtgewicht des gefriergetrockneten Pulvers, umfasst.

16. Produkt nach einem der Ansprüche 13 oder 14, **dadurch gekennzeichnet, dass** das gefriergetrocknete Pulver die Mikrovesikel und/oder Exosomen in einer Menge zwischen 20 und 60 Gew.-% und das mindestens eine Kryo-Schutzmittel in einer Menge zwischen 80 und 40 Gew.-% enthält.

17. Produkt nach Anspruch 13, **dadurch gekennzeichnet, dass** die Mikrovesikel und/oder Exosomen Nanopartikel umfassen, die mit einer biologisch aktiven Substanz beladen sind.

18. Produkt nach Anspruch 17, **dadurch gekennzeichnet, dass** die biologisch aktive Substanz ausgewählt ist aus der Gruppe bestehend aus kardiovaskulären Arzneimitteln, Arzneimitteln des zentralen und peripheren Nervensystems, schmerzstillenden Arzneimitteln, antimikrobiellen Arzneimitteln, Chemotherapie-Arzneimitteln, Anti-Tumor-Arzneimitteln, Anti-Tumor-Immuntherapie-Arzneimitteln, Impfstoffen, Hormonen, Peptid-und Protein-Arzneimitteln, Vitaminen, Pflanzenextrakten und Phytokomplexen.

19. Pharmazeutische Zusammensetzung, die eine biologisch aktive Substanz und mindestens einen pharmazeutisch geeigneten Hilfsstoff umfasst, wobei die biologisch aktive Substanz in Nanopartikel geladen ist, die in gefriergetrockneten Mikrovesikeln und/oder Exosomen mit einem Durchmesser zwischen 20 und 5000 nm und einem durchschnittlichen Durchmesser zwischen 50 und 200 nm enthalten sind; **dadurch gekennzeichnet, dass** die gefriergetrockneten Mikrovesikel und/oder Exosomen in einem Produkt in Form eines gefriergetrockneten Pulvers nach einem der Ansprüche 13 bis 18 zusammen mit mindestens einem Kryo-Schutzmittel enthalten sind.

20. Pharmazeutische Zusammensetzung nach Anspruch 19, **dadurch gekennzeichnet, dass** die biologisch aktive Substanz ausgewählt ist aus der Gruppe bestehend aus kardiovaskulären Arzneimitteln, Arzneimitteln des zentralen und peripheren Nervensystems, schmerzstillenden Arzneimitteln, antimikrobiellen Arzneimitteln, Chemotherapie-Arzneimitteln, Anti-Tumor-Arzneimitteln, Anti-Tumor-Immuntherapie-Arzneimitteln, Impfstoffen, Hormonen, Peptid- und Protein-Arzneimitteln, Vitaminen, Pflanzenextrakten und Phytokomplexen.

21. Pharmazeutische Zusammensetzung nach Anspruch 20, **dadurch gekennzeichnet, dass** die kardiovaskulären Arzneimittel ausgewählt sind aus der Gruppe bestehend aus Antihypertensiva, Antiarrhythmika und Diuretika.

22. Pharmazeutische Zusammensetzung nach Anspruch 20, **dadurch gekennzeichnet, dass** die Arzneimittel des zentralen und peripheren Nervensystems aus der Gruppe ausgewählt sind, die aus Anästhetika, Hypnotika, Sedativa, Anxiolytika und Anti-Parkinson-Mitteln besteht.

23. Pharmazeutische Zusammensetzung nach Anspruch 20, **dadurch gekennzeichnet, dass** die schmerzstillenden Arzneimittel aus der Gruppe ausgewählt sind, die aus entzündungshemmenden und analgetischen Arzneimitteln besteht.

24. Pharmazeutische Zusammensetzung nach Anspruch 20, **dadurch gekennzeichnet, dass** die antimikrobiellen Arzneimittel aus der Gruppe ausgewählt sind, die aus sulfamidischen Arzneimitteln, Antibiotika, antiviralen Arzneimitteln und Antimykotika besteht.

25. Pharmazeutische Zusammensetzung nach einem der vorhergehenden Ansprüche 19 bis 24 zur Verwendung bei der Behandlung von Arthrose, Herz-Kreislauf-Erkrankungen, Atemwegserkrankungen, neurologischen Erkrankungen, Schmerzen, Knochenmarkversagen, Diabetes und Krebs.

26. Kosmetische oder kosmezeutische Zusammensetzung, die eine biologisch aktive Substanz und mindestens einen kosmetisch geeigneten Hilfsstoff enthält, wobei die biologisch aktive Substanz in Nanopartikel geladen ist, die in gefriergetrockneten Mikrovesikeln und/oder Exosomen mit einem Durchmesser zwischen 20 und 5000 nm und einem mittleren Durchmesser zwischen 50 und 200 nm enthalten sind, **dadurch gekennzeichnet, dass** die gefriergetrockneten Mikrovesikel und/oder Exosomen in einem gefriergetrockneten Pulver nach einem der Ansprüche 13 bis 18 zusammen mit mindestens einem Kryo-Schutzmittel enthalten sind.

27. Kosmetische oder kosmezeutische Zusammensetzung nach Anspruch 26 zur Verwendung bei der Anti-Aging-Behandlung der Haut.

28. Pharmazeutische Zusammensetzung, die Mikrovesikel und/oder Exosomen mit einem Durchmesser zwischen 20 und 5000 nm und einem mittleren Durchmesser zwischen 50 und 200 nm und mindestens einen pharmazeutisch akzeptablen Hilfsstoff für die topische Behandlung von Verletzungen und akuten und chronischen Wunden umfasst, **dadurch gekennzeichnet, dass** die gefriergetrockneten Mikrovesikel und/oder Exosomen in einem gefriergetrockneten Pulver nach einem der Ansprüche 13 bis 18 zusammen mit mindestens einem Kryo-Schutzmittel enthalten sind.

29. Pharmazeutische Zusammensetzung nach Anspruch 28, wobei der pharmazeutisch annehmbare Hilfsstoff eine Membran für fortgeschrittene Verbände umfasst, und die Membran für fortgeschrittene Verbände wahlweise Natriumalginat, Calciumalginat, Cellulosederivate (vorzugsweise Carboxymethylcellulose), Ester der Hyaluronsäure, Seidenproteine und Vliesstoff umfasst.

## Revendications

1. Procédé de production de microvésicules et/ou d'exosomes, **caractérisé en ce qu'**il comprend les étapes suivantes :
(i) la collecte d'un fluide biologique comprenant des microvésicules et/ou exosomes,
(ii) la dialyse ou l'ultrafiltration dudit fluide biologique au moyen d'une membrane présentant une valeur seuil (coupure) inférieure ou égale à 5 000 Dalton et un fluide de dialyse ou d'ultrafiltration,
(iii) l'ajout d'un cryoprotecteur à la solution dialysée ou ultrafiltrée obtenue à partir de l'étape (ii), et
(iv) la lyophilisation de la solution résultant de l'étape (iii) obtenant une poudre lyophilisée comprenant des microvésicules et/ou exosomes dans une quantité supérieure ou égale à 20 % p/p par rapport au poids total de ladite poudre lyophilisée ; et au moins un cryoprotecteur dans une quantité inférieure ou égale à 80 % p/p.

2. Procédé de production de microvésicules et/ou d'exosomes selon la revendication 1, **caractérisé en ce que** ledit fluide biologique est un surnageant de cellules en culture, de préférence collectées à partir de cellules en culture dans un milieu de culture, en particulier un milieu de culture minimal, pendant au moins 4 heures.

3. Procédé de production de microvésicules et/ou d'exosomes selon la revendication 2, **caractérisé en ce que** lesdites cellules sont des cellules souches et en particulier des cellules souches mésenchymateuses.

4. Procédé de production de microvésicules et/ou d'exosomes selon la revendication 3, **caractérisé en ce que** lesdites cellules souches sont des cellules souches mésenchymateuses isolées à partir d'un tissu adipeux par lavage avec une solution tampon et digestion enzymatique.

5. Procédé de production de microvésicules et/ou d'exosomes selon l'une quelconque des revendications 2 à 4, **caractérisé en ce que** lesdites cellules sont mises en culture dans un milieu de culture contenant des nanoparticules chargées d'une substance biologiquement active.

6. Procédé de production de microvésicules et/ou d'exosomes selon la revendication 1, **caractérisé en ce que** ledit fluide biologique est un fluide corporel.

7. Procédé de production de microvésicules et/ou d'exosomes selon la revendication 1, **caractérisé en ce que** ledit fluide de dialyse ou d'ultrafiltration est de l'eau ou une solution tampon avec une concentration de sels inférieure à la concentration de sels du fluide biologique.

8. Procédé de production de microvésicules et/ou d'exosomes selon la revendication 1, **caractérisé en ce que** ledit fluide de dialyse comprend un polyoxyalkylène présentant un poids moléculaire moyen supérieur ou égal à 100 g/mol, par exemple un polyéthylène glycol (PEG) présentant un poids moléculaire moyen supérieur ou égal à 100 g/mol, de préférence supérieure ou égale à 300 g/mol, plus préférentiellement supérieure ou égale à 500 g/mol, et inférieure ou égale à 4 000 g/mol, de préférence inférieure ou égale à 3 000 g/mol, plus préférentiellement inférieure ou égale à 2 500 g/mol.

9. Procédé de production de microvésicules et/ou d'exosomes selon la revendication 1, **caractérisé en ce que** ledit cryoprotecteur est sélectionné dans le groupe consistant en sucres, acides aminés, polyols ou mélanges de ceux-ci.

10. Procédé de production de microvésicules et/ou d'exosomes selon la revendication 9, **caractérisé en ce que** ledit cryoprotecteur est sélectionné dans le groupe consistant en mannitol, lactose, sucrose, tréhalose, sorbitol, glucose, maltose, fructose, raffinose, arginine, alanine, glycine, histidine, éthylène glycol, 1,3-propanediol, glycérol, cyclodextrines et mélanges de ceux-ci.

11. Procédé de production de microvésicules et/ou d'exosomes selon la revendication 10, **caractérisé en ce que** ledit cryoprotecteur est sélectionné dans le groupe consistant en mannitol, glycine ou mélanges de ceux-ci.

12. Procédé de production de microvésicules et/ou d'exosomes selon la revendication 1, **caractérisé en ce que** l'étape de lyophilisation (iv) comprend les étapes suivantes :
a) la congélation de la solution dialysée ou ultrafiltrée obtenue à partir de l'étape (iii) fonctionnant à une température inférieure à -50 °C, de préférence inférieure à -70 °C,
b) la sublimation de la solution congelée sous vide dans un lyophilisateur.

13. Produit sous forme de poudre lyophilisée obtenue par le procédé selon l'une quelconque des revendications précédentes, comprenant des microvésicules et/ou exosomes présentant un diamètre compris entre 20 et 5 000 nm et un diamètre moyen compris entre 50 et 200 nm, et au moins un cryoprotecteur ; dans lequel ladite poudre lyophilisée comprend lesdits microvésicules et/ou exosomes dans une quantité supérieure ou égale à 20 % p/p par rapport au poids total de ladite poudre lyophilisée ; et ledit au moins un cryoprotecteur dans une quantité inférieure ou égale à 80 % p/p.

14. Produit selon la revendication 13, **caractérisé en ce que** ledit cryoprotecteur est sélectionné dans le groupe consistant en sucres, acides aminés, polyols ou mélanges de ceux-ci.

15. Produit selon l'une quelconque des revendications 13 ou 14, **caractérisé en ce que** ladite poudre lyophilisée comprend lesdits microvésicules et/ou exosomes dans une quantité supérieure ou égale à 30 % p/p et de préférence supérieure ou égale à 40 % p/p par rapport au poids total de ladite poudre lyophilisée ; et ledit au moins un cryoprotecteur dans une quantité inférieure ou égale à 70 % p/p, de préférence inférieure ou égale à 60 % p/p par rapport au poids total de ladite poudre lyophilisée.

16. Produit selon l'une quelconque des revendications 13 ou 14, **caractérisé en ce que** ladite poudre lyophilisée contient lesdits microvésicules et/ou exosomes dans une quantité comprise entre 20 % et 60 % p/p, et ledit au moins un cryoprotecteur dans une quantité comprise entre 80 % et 40 % p/p.

17. Produit selon la revendication 13, **caractérisé en ce que** lesdits microvésicules et/ou exosomes comprennent des nanoparticules chargées d'une substance biologiquement active.

18. Produit selon la revendication 17, **caractérisé en ce que** ladite substance biologiquement active est sélectionnée dans le groupe consistant en médicaments cardiovasculaires, médicaments du système nerveux central et périphérique, médicaments anti-douleur, médicaments antimicrobiens, médicaments de chimiothérapie, médicaments antitumoraux, médicaments d'immunothérapie antitumoraux, vaccins, hormones, médicaments peptidiques et protéiniques, vitamines, extraits de plante et phytocomplexes.

19. Composition pharmaceutique comprenant une substance biologiquement active et au moins un excipient pharmaceutiquement acceptable, ladite substance biologiquement active étant chargée dans des nanoparticules comprises dans les microvésicules et/ou exosomes lyophilisés présentant un diamètre compris entre 20 et 5 000 nm et un diamètre moyen compris entre 50 et 200 nm ; **caractérisée en ce que** lesdits microvésicules et/ou exosomes lyophilisés sont compris dans un produit sous forme de poudre lyophilisée selon l'une quelconque des revendications 13 à 18 conjointement avec au moins un cryoprotecteur.

20. Composition pharmaceutique selon la revendication 19, **caractérisée en ce que** ladite substance biologiquement active est sélectionnée dans le groupe consistant en médicaments cardiovasculaires, médicaments du système nerveux central et périphérique, médicaments anti-douleur, médicaments antimicrobiens, médicaments de chimiothérapie, médicaments antitumoraux, médicaments d'immunothérapie antitumoraux, vaccins, hormones, médicaments peptidiques et protéiniques, vitamines, extraits de plante et phytocomplexes.

21. Composition pharmaceutique selon la revendication 20, **caractérisée en ce que** lesdits médicaments cardiovasculaires sont sélectionnés dans le groupe consistant en antihypertenseurs, médicaments antiarythmiques et diurétiques.

22. Composition pharmaceutique selon la revendication 20, **caractérisée en ce que** lesdits médicaments du système nerveux central et périphérique sont sélectionnés dans le groupe consistant en médicaments anesthésiques, hypnotiques, sédatifs, anxiolytiques et anti-Parkinson.

23. Composition pharmaceutique selon la revendication 20, **caractérisée en ce que** lesdits médicaments anti-douleur sont sélectionnés dans le groupe consistant en médicaments anti-inflammatoires et analgésiques.

24. Composition pharmaceutique selon la revendication 20, **caractérisée en ce que** lesdits médicaments antimicrobiens sont sélectionnés dans le groupe consistant en médicaments sulfamidiques, antibiotiques, antiviraux et médicaments antifongiques.

25. Composition pharmaceutique selon l'une quelconque des revendications précédentes 19 à 24 pour son utilisation dans le traitement de l'arthrose, des maladies cardiovasculaires, des maladies respiratoires, des maladies neurologiques, de la douleur, de l'insuffisance médullaire, du diabète et du cancer.

26. Composition cosmétique ou cosméceutique comprenant une substance biologiquement active et au moins un excipient cosmétiquement acceptable, ladite substance biologiquement active étant chargée dans des nanoparticules comprises dans les microvésicules et/ou exosomes lyophilisés présentant un diamètre compris entre 20 et 5 000 nm et un diamètre moyen compris entre 50 et 200 nm ; **caractérisée en ce que** lesdits microvésicules et/ou exosomes lyophilisés sont compris dans une poudre lyophilisée selon l'une quelconque des revendications 13 à 18 conjointement avec au moins un cryoprotecteur.

27. Composition cosmétique ou cosméceutique selon la revendication 26 pour son utilisation dans le traitement antivieillissement cutané.

28. Composition pharmaceutique comprenant des microvésicules et/ou exosomes présentant un diamètre compris entre 20 et 5 000 nm et un diamètre moyen compris entre 50 et 200 nm et au moins un excipient pharmaceutiquement acceptable pour le traitement topique de blessures et plaies aigues et chroniques ; **caractérisée en ce que** lesdits microvésicules et/ou exosomes lyophilisés sont compris dans une poudre lyophilisée selon l'une quelconque des revendications 13 à 18 conjointement avec au moins un cryoprotecteur.

29. Composition pharmaceutique selon la revendication 28, dans laquelle ledit excipient pharmaceutiquement acceptable comprend une membrane pour des pansements perfectionnés, et ladite membrane pour perfectionnés comprend facultativement de l'alginate de sodium, de l'alginate de calcium, des dérivés de cellulose (de préférence de la carboxyméthylcellulose), des esters d'acide hyaluronique, des protéines de soie et un tissu non-tissé.
